(19) Europäisches Patentamt
European Patent Office
Office européen des brevets

(11) Veröffentlichungsnummer : **0 233 483 B1**

# (12) EUROPÄISCHE PATENTSCHRIFT

(45) Veröffentlichungstag der Patentschrift : **03.04.91 Patentblatt 91/14**

(21) Anmeldenummer : **87100598.9**

(22) Anmeldetag : **19.01.87**

(51) Int. Cl.$^5$ : **C07D 498/04**, C07D 519/00, A61K 31/55, // (C07D498/04, 267:00, 209:00), (C07D519/00, 498:00, 471:00)

(54) **Pyrrolo[1,2-a][4,1]benzoxazepine, Verfahren zur ihrer Herstellung, pharmazeutische Präparate enthaltend diese Verbindungen, sowie therapeutische Verwendung.**

Die Akte enthält technische Angaben, die nach dem Eingang der Anmeldung eingereicht wurden und die nicht in dieser Patentschrift enthalten sind.

(30) Priorität : **21.01.86 US 821110**

(43) Veröffentlichungstag der Anmeldung : **26.08.87 Patentblatt 87/35**

(45) Bekanntmachung des Hinweises auf die Patenterteilung : **03.04.91 Patentblatt 91/14**

(84) Benannte Vertragsstaaten : **AT BE CH DE ES FR GB GR IT LI LU NL SE**

(56) Entgegenhaltungen :
**EP-A- 0 144 729**
**US-A- 4 029 672**
**US-A- 4 053 599**
**Farmaco, Ed. Sci. (1983) 38, 893-903**

(73) Patentinhaber : **CIBA-GEIGY AG**
**Klybeckstrasse 141**
**CH-4002 Basel (CH)**

(72) Erfinder : **Wasley, Jan William Francis**
**55 Floral Street**
**Chatham New Jersey 07928 (US)**
Erfinder : **Norman, Jon**
**249 Evergreen Court**
**Mountainside New Jersey 07092 (US)**

(74) Vertreter : **Zumstein, Fritz, Dr. et al**
**Bräuhausstrasse 4**
**W-8000 München 2 (DE)**

**EP 0 233 483 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue substituierte Pyrrolo[1,2-a][4,1]benzoxazepine, insbesondere an C-4 substituierte Pyrrolo[1,2-a][4,1]benzoxazepine, deren Herstellung, sowie pharmazeutische Präparate, die diese neuen Verbindungen enthalten und auch ihre Verwendung.

Aus Farmaco, Ed. Sci. (1983) 38, 893-903 sind 4H,6H-Pyrrolo[1,2-a][4,1]benzoxazepine bekannt, die an C-4 unsubstituiert sind und ZNS-wirksam sind und in US-A-4 096 672 und US-A-4 053 599 werden an C-4 substitierte, strukturisomere Pyrrolo[2,1-c][1,4]benzoxazepine beschrieben, welche analgetische, antikonvulsive und sedative Eigenschaften bzw. antihypertensive Eigenschaften aufweisen. Auch in EP-A-0 144 729 werden an C-4 substituierte, strukturisomere Pyrrolo[2,1-c][1,4]benzoxazepine beschrieben, welche antipsychotische und analgetische Eigenschaften besitzen.

Ueberraschenderweise wurde nun gefunden, dass die neuen 4-substituierten 4H,6H-Pyrrolo[1,2-a][4,1]benzoxazepine der allgemeinen Formel

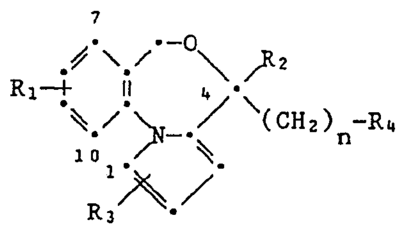

(I),

worin n für eine ganze Zahl 1 oder 2 steht; $R_1$ Wasserstoff, Niederalkyl, Halogen, Trifluormethyl, Hydroxy oder Niederalkoxy bedeutet; $R_2$ Wasserstoff oder Niederalkyl ist; $R_3$ Wasserstoff, Niederalkyl oder Halogen bedeutet; $R_4$ N-Niederalkyl piperazino oder unsubstituiertes oder durch Halogen oder Niederalkyl im Phenylrest substituiertes N-Phenylpiperazino, Piperidino oder substituiertes Piperidino ausgewählt aus

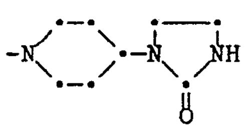

,

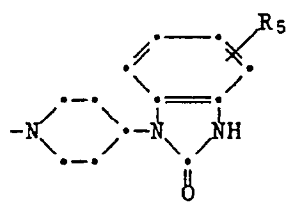

,

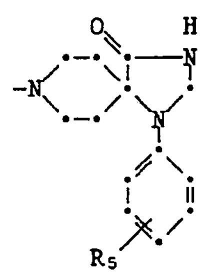

,

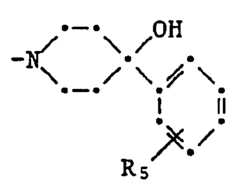

und

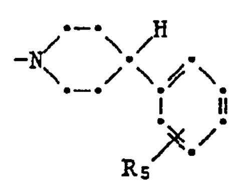

worin $R_5$ Wasserstoff, Halogen oder Niederalkyl bedeutet, darstellt; und Salze davon gastrointestinalsekretionshemmende Wirksamkeit, insbesondere anti-diarrhötische Wirksamkeit aufweisen.

Bevorzugt sind Verbindungen der allgemeinen Formel II

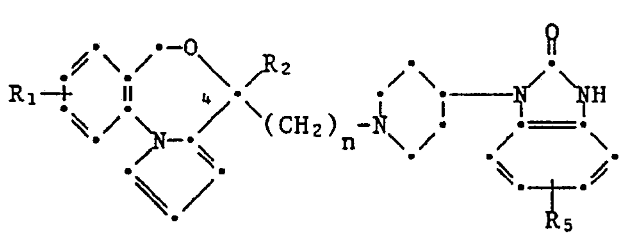

(II),

worin $R_1$ Wasserstoff, Niederalkyl, Halogen, Trifluormethyl, Hydroxy oder Niederalkoxy bedeutet ; $R_2$ Wasserstoff oder Niederalkyl ; n eine ganze Zahl von 1 bis 2 darstellt ; und $R_5$ Wasserstoff, Halogen oder Niederalkyl bedeutet ; und pharmazeutisch annehmbare Salze davon.

Bevorzugt sind weiter Verbindungen der Formel II, worin n für die ganze Zahl 1 oder 2 steht ; $R_2$ geradkettiges Niederalkyl mit 1 bis 4 Kohlenstoffatomen bedeutet ; $R_1$ und $R_5$ Wasserstoff oder Halogen bedeuten; und pharmazeutisch annehmbare Salze davon.

Besonders bevorzugt sind Verbindungen der Formel II, worin $R_1$ und $R_5$ Wasserstoff, $R_2$ Methyl oder Ethyl, und n die Zahl 1 bedeuten, und pharmazeutisch annehmbare Salze davon.

Die hier verwendeten allgemeinen Definitionen haben im Rahmen der vorliegenden Erfindung die folgende Bedeutung.

Halogen ist vorzugsweise Chlor und Fluor aber auch Brom oder Jod.

Der Ausdruck "nieder" definiert in den oben oder nachfolgend genannten organischen Resten oder Verbindungen solche mit höchstens 7, vorzugsweise 4, insbesondere 1 oder 2 Kohlenstoffatomen.

Eine Niederalkylgruppe enthält 1 bis 7 Kohlenstoffatome, insbesondere 1 bis 4 Kohlenstoffatome, ist vorzugsweise geradkettig und bedeutet Methyl, Ethyl, Propyl oder Butyl, insbesondere Methyl.

Niederalkoxy ist vorzugsweise geradkettig und bedeutet z.B. Methoxy, Ethoxy, Propoxy, insbesondere Methoxy.

Phenyl ist unsubstituiert oder kann durch Niederalkyl oder Halogen monosubstituiert sein.

Pharmazeutisch annehmbare Salze sind Säureadditionssalze, die vorzugsweise von therapeutisch verwendbaren anorganischen oder organischen Säuren abgeleitet sind, wie beispielsweise von starken Mineralsäuren, z.B. Halogenwasserstoffsäuren, wie Salzsäure oder Bromwasserstoffsäure ; Schwefel-, Phosphor- oder Salpetersäure ; aliphatischen oder aromatischen Sulfonsäuren, z.B. Methansulfonsäure, Ethansulfonsäure, Hydroxyäthansulfonsäure, Benzolsulfonsäure, p-Toluolsulfonsäure oder Naphthalensulfonsäure ; aliphatischen oder aromatischen Carbonsäuren, z.B. Essig-, Propion-, Bernstein-, Glykol-, Milch-, Aepfel-, Wein-, Glucon-, Zitronen-, Malein-, Fumar-, Pyruvin-, Phenylessig-, Benzol-, 4-Aminobenzol-, Anthranil-, 4-Hydroxybenzol-, Salicyl-, 4-Aminosalicyl-, Pamoenikotinsäure, oder von Askorbinsäure.

Die Verbindungen der vorliegenden Erfindong zeigen wertvolle pharmakologische Eigenschaften wie z.B. gastrointestinalsekretionshemmende Wirksamkeit, insbesondere anti-diarrhöische Wirksamkeit, sie antagonisieren, inter alia Calmodulinwirkung bei Säugern.

Die obengenannten Eigenschaften können durch in vitro und in vivo Versuche, vorzugsweise an Säugetieren, wie Mäusen, Ratten oder Hunden, als Testobjekte, nachgewiesen werden. Die erfindungsgemässen Verbindungen können enteral, parenteral, vorzugsweise oral, oder subkutan, intravenös oder intraperitoneal, z.B. durch Gelatine-Kapseln oder in Form von wässrigen Lösungen oder Suspensionen, verabreicht werden. Die verwendete Dosis kann in einem Bereich von ungefähr 0,10 und 100 mg/kg/Tag, vorzugsweise ungefähr 0,50 und 50 mg/kg/Tag, vorzugsweise zwischen 5 und 30 mg/kg/Tag liegen.

Die verwendbare Dosis in vitro kann ungefähr zwischen $10^{-4}$ und $10^{-8}$ M Konzentration, vorzugsweise ungefähr zwischen $10^{-5}$ und $10^{-7}$ M liegen.

Die Hemmung von Calmodulinwirkung, gezeigt durch die sekretionshemmende Wirksamkeit der neuen erfindungsgemässen Verbindungen kann in vitro durch die Emittlung der Messwerte durch die Camoldulinhemmung induzierte Aktivierung der Rinder c-AMP Phosphodiesterase, gemäss der Methode von J.A. Norman et al. "Mol. Pharmacology 16, 1089 (1979) nachgewiesen werden.

Als Beispiel für die Illustration der Erfindung sei das 1,3-Dihydro-1-{1-[(4-methyl-4H,6H-pyrrolo[1,2-a][4,1]benzoxazepin-4-yl)-methyl]-4-piperidinyl}-2H-benzimidazol-2-on-maleat gennant, welches durch die Calmodulinhemmung die Aktivierung der Phosphodiesterase in vitro bei 50% in Konzentration von ungefähr 3 $\times 10^{-6}$ Molar induziert.

Die antidiarrhöische Wirksamkeit der erfindungsgemässe Verbindungen der Formel I kann durch die Messung der Hemmung der Diarrhöe, welche bei den Ratten durch intraperitoneal verabreichtes Prostagladin $E_2$ induziert wurde, nachgewiesen werden.

Der Test wird wie folgt durchgeführt : Für den Versuch wurden weibliche Sprague-Dawley Ratten mit einem Körpergewicht von 200-250 g verwendet. Die Tiere kommen 18 Stunden nüchtern in den Versuch. Die Testverbindungen werden in Dimethylsulfoxid mit Wasser zu einer 30-50% Dimethylsulfoxid-Konzentration verdünnt und den Ratten oral bei Magenwäsche volumenmässig 1 ml/kg verabreicht. Dreissig oder sechzig Minuten später wird das Prostagladin $E_2$ (150 µg) im Volumen von 0,5 ml Wasser verabreicht. Die Verminderung der Kotausscheidung im Vergleich zu den nicht behandelten Tieren wird für eine Periode von 30 Minuten nach der Verabreichung von Prostagladin $E_{21}$ gemessen.

Als Beispiel für die Illustration der Erfindung sei das 1,3-Dihydro-1-{1-[(4-methyl-4H,6H-pyrrolo[1,2-a][4,1]benzoxazepin-4-yl)-methyl]-4-piperidinyl}-2H-benzimidazol-2-on-maleat genannt, welches die beschriebene Wirkung bei einer Dosis von 10 mg/kg p.o. bei Ratte zeigt.

Die Hemmung der Magensäuresekretion, als Indikator der Hemmung der Bildung von Magengeschwüren, wurde in vitro an parietalen Zellen der Rattenmägen wie folgt bestimmt :

Etwa 1 × $10^6$ parietale Zellen (18-22% angereichert) werden in einer Glukose enthaltenden Salzlösung, BSA (Rinder Serum Albumin), 0,1 µCi von $^{14}C$-Aminopyrin, Histamin (1 mM), Isobutyl-methylxanthin (0,1 mM) inkubiert und die Test-Verbindung wird unter 95% Sauerstoff bei 37°C während 40 Minuten getestet. Die Zellen werden dann gewaschen, mit destilliertem Wasser getrennt und bei Scintillation-Spektrometrie analysiert. Eine Abnahme in der Ansammlung von $^{14}C$-Aminopyrin in den Zellen zeigt indirekt die Höhe der Magensäurehemmung.

Als Beispiel für die Illustration der Erfindung sei das 1,3-Dihydro-1-{1-[(4-methyl-4H,6H-pyrrolo[1,2-a][4,1]benzoxazepin-4-yl)-methyl]-4-piperidinyl}-2H-benzimidazol-2-on-maleat genannt, welches Magensäuresekretion-Hemmung in den parietalen Zellen der Rattenmägen mit $IC_{50}$ bei etwa 3 × $10^{-7}$ M zeigt.

Die erfindungsgemässen Verbindungen der Formel I können als Gastrointestinalwirkstoffe, wie gastrointestinale Antisekretionsmittel, z.B. zur Behandlung der Diarrhöe, Ulcera, übermässiger Magensäuresekretion, inflammatorischen Darmkrankheiten und Dehydratation verwendet werden.

Die erfindungsgemässen Verbindungen der Formel I oder II und ihre Salze können hergestellt werden, indem man

a) ein Amin der Formel III

$$R_4\text{-H} \quad (III)$$

worin $R_4$ die oben angegebene Bedeutung hat und H an dem Stickstoffatom der Gruppe $R_4$ gebunden ist, mit einer Verbindung der Formel IV

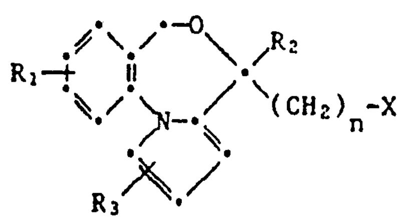

(IV),

worin n, $R_1$, $R_2$ und $R_3$ die unter Formel I angegebene Bedeutung haben und X eine reaktionsfähige veresterte Hydroxygruppe bedeutet, kondensiert, oder mit einer Verbindung der Formel V

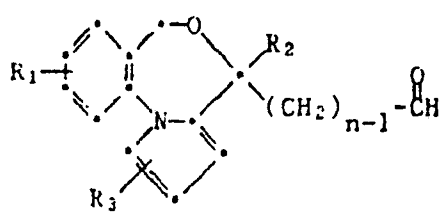

(V),

worin n, $R_1$, $R_2$ und $R_3$ die unter Formel I angegebene Bedeutung haben, unter Bedingungen einer reduktiven Aminierung kondensiert ; oder

b) eine Verbindung der Formel VI

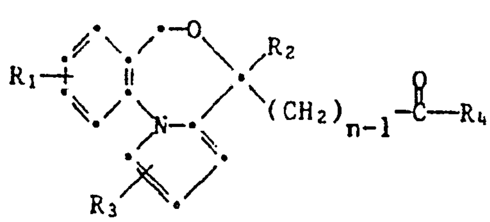

(VI),

worin $R_1$, $R_2$, $R_3$, $R_4$ und n die unter Formel I angegebene Bedeutung haben, redoziert und, wenn erwünscht, in allen diesen Verfahren eine störende reaktionsfähige Gruppe vorübergehend schützt, und die erhaltene Verbindung der Formel I isoliert ; und, wenn erwünscht, eine erhaltene Verbindung der Formel I in eine andere erfindungsgemässe Verbindung der Formel I umwandelt, und/oder, wenn erwünscht, eine erhaltene Verbindung der Formel I in ihr Salz oder ein erhaltenes Salz in die freie Verbindung oder in ein anderes Salz überführt, und/oder wenn erwünscht, ein erhaltenes Gemisch von Isomeren oder Razematen in die einzelnen Isomeren oder Razemate auftrennt, und/oder wenn erwünscht, erhaltene Razemate in die optischen Antipoden trennt.

In den genannten Ausgangsstoffen der oben beschriebenen Verfahren steht die reaktionsfähige veresterte Hydroxygruppe für eine durch eine starke Säure, insbesondere durch eine anorganische Säure, wie Halogenwasserstoffsäure, in erster Linie wie Chlorwasserstoff-, Bromwasserstoff- oder Jodwasserstoffsäure, oder Schwefelsäure, oder durch eine starke organische Säore, insbesondere Sulfonsäure, wie eine aliphatische oder aromatische Sulfonsäure, z.B. Methansulfonsäure, 4-Methylphenylsulfonsäure oder 4-Bromphenylsulfonsäure veresterte Hydroxygruppe.

In den Ausgangsstoffen und Zwischenprodukten, die zu den erfindungsgemässen Verbindungen auf eine hierin beschriebene Art und Weise umgesetzt werden, können anwesende funktionelle Gruppen, z.B. Formyl-, Carboxy-, Amino- und Hydroxygruppen durch übliche Schutzgruppen, die in der präparativen organischen Chemie bekannt sind, geschützt werden. Geschützte Formyl-, Carboxy-, Amino- und Hydroxygruppen sind solche, die unter milden Bedingungen in die freien Formyl-, Carboxy-, Amino-, oder Hydroxygruppen umgesetzt werden können, ohne dass das Molekülgerüst zerstört oder andere, unerwünschte Nebenreaktionen stattfinden werden. Die Notwendigkeit zur Anwendung und die Auswahl der Schutzgruppen für eine spezielle Reaktion ist dem Fachmann bekannt und ist abhängig von der zu schützenden Gruppe (Formyl-, Carboxy-, Aminogruppe usw.), von der Struktur und Stabilität des Moleküls, von dem der Substituent ein Teil ist, und von den Reaktionsbedingungen.

Bekannte Schutzgruppen, die diese Bedingungen erfüllen, sowie ihre Einführung und ihre Abspaltung werden z.B. beschrieben in J.F.W. McOmie, "Protective Groups in Organic Chemistry", Plenum Press, London/New York, 1973, in T.W. Greene, "Protective Groups in Organic Synthesis", Wiley, New York 1981 ; und auch in "The Peptides", Vol. 1, Schroeder und Luebke, Academic Press, London/New York, 1965 ; ebenso wie in Houben-Weyl, "Methoden der Organischen Chemie", Band 15/1, Georg Thieme-Verlag, Stuttgart, 1974.

Die Herstellung der erfindungsgemässen Verbindungen nach dem Verfahren a) umfassend die N-Alkylierung von einem Amin der Formel III mit einer Verbindung der Formel IV wird nach bekannten Methoden durchgeführt, gewöhnlich in einem inerten Lösungsmittel oder einem Gemisch davon und, wenn notwendig, unter Kühlung oder Erwärmung, z.B. in einem Temperaturbereich zwischen von etwa –20°C bis etwa 150°C, und/oder in einer Inertgasatmosphäre durchgeführt.

Die Reaktion wird vorzugsweise in Gegenwart von einer Base, wie einer anorganischen Base, z.B. eines Alkalimetall- oder Erdalkalimetall-carbonats, -hydrids oder -hydroxids, oder in Gegenwart von einer organischen Base, wie z.B. eines tertiären Amins, wie Triethylamin oder Pyridin, durchgeführt.

Die Herstellung der erfindungsgemässen Verbindungen nach dem Verfahren a) betreffend die Kondensation eines Amins der Formel III mit einem Aldehyd der Formel V bei reduktiver N-Alkylierung wird in an sich bekannter Weise, wie z.B. bei katalytischer Hydrogenation mit katalytisch aktiviertem Wasserstoff, wobei Edelmetallkatalystoren verwendet werden, welche Platin, Palladium oder Nickel enthalten, oder mit chemischen Reduktionsmitteln, wie Verbindungen oder Komplexen von Leichtmetallhydriden, insbesondere Alkalimetallcyanborhydrid, wie Natriumcyanborhydrid, durchgeführt. Bei Verwendung von einem Alkalimetallcyanborhydrid wird vorzogsweise in Gegenwart von einem inerten Lösungsmittel, wie z.B. Methanol oder Acetonitryl, insbesondere in Gegenwart einer Säure, z.B. Chlorwasserstoff- oder Essigsäure, gearbeitet.

Die Verfahrensvariante a) wird insbesondere für die Herstellung von erfindungsgemässen Verbindungen der Formel I, worin eine fonktionelle Amidgruppe vorhanden ist, verwendet.

Die Herstellung der erfindungsgemässen Verbindungen der Formel I nach dem Verfahren b) erfolgt mittels geeignet Reduktionsmittel in an sich bekannter Weise. Solche sind in erster Linie HydridReduktionsmittel, wie komplexe Metallhydride, z.B. Lithiumaluminiumhydrid, oder Borhydride, die man üblicherweise in einem inerten Lösungsmittel, wie Tetrahydrofuran oder Diethylether, anwendet, wobei man insbesondere in einem Temperaturbereich von Zimmertemperatur bis nahe dem Siedepunkt des Lösungsmittels arbeitet.

Die Ausgangsverbindungen der Formel III sind bekannt, oder können nach bekannten Methoden hergestellt werden.

Die Ausgangsverbindungen der Formel IV und V werden nach bekannten Methoden hergestellt.

Ein Ester der entsprechend substituierten Anthranilsäure wird mit einem 2,5-Diniederalkoxytetrahydrofuran, z.B. in Essigsäure erhitzt und zu dem o-(1-Pyrrolyl)benzoesäure-Ester, z.B. Niederalkylester, kondensiert, welcher nachher, z.B. mit Lithiumaluminiumhydrid in einem inerten Lösungsmittel, zu dem entsprechend substituierten o-(1-Pyrrolyl)-benzylalkohol der Formel VII

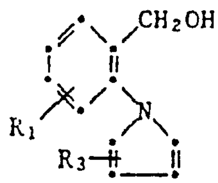

(VII),

worin $R_1$ und $R_3$ die unter Formel I angegebene Bedeutung haben, reduziert wird.

Die Kondensation einer Verbindung der Formel VII mit einer Verbindung der Formel VIII

$$R_2-\underset{\underset{O}{\|}}{C}-(CH_2)_{n-1}-COOH \qquad (VIII),$$

worin n und $R_2$ die unter Formel I angegebene Bedeutung hat, oder einem Ester davon, z.B. Niederalkylester, vorzugsweise in Gegenwart einer starken Base, wie z.B. Butyllithium oder Lithiumdiisopropylamid, oder in Gegenwart einer Säure, z.B. Bromwasserstoffsäure, erhält man eine Verbindung der Formel IX

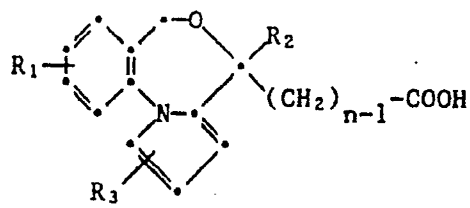

(IX),

oder einen Ester davon, z.B. Nideralkylester, worin n, $R_1$, $R_2$ und $R_3$ die unter Formel I angegebene Bedeutung haben.

Die Ausgangsverbindungen der Formel V erhält man durch Reduktion eines Esters von einer Verbindung der Formel IX mit einem selektiven chemischen Reduktionsmittel, z.B. Diisobutylaluminiumhydrid.

Die Ausgangsverbindungen der Formel IV, worin X Hydroxy bedeutet, erhält man durch Reduktion einer Verbindung der Formel IX mit einem Redutitionsmittel, welches für die Reduktion einer Carbonsäure oder eines Esters des entsprechenden Alkohols geeignet ist, wie z.B. Diboran, Natrium-bis-(2-methoxyethoxy)-aluminiumhydroxid oder Lithiumaluminiumhydrid.

Die Ausgangsverbindungen der Formel IV, worin X Hydroxy bedeutet, können in die entsprechenden Verbindungen der Formel IV, worin X verestertes Hydroxy bedeutet, in an sich bekannter Weise überführt werden. So können z.B. Verbindungen der Formel IV, worin Halogen, wie z.B. Chlor oder Brom, bedeutet, durch Behandeln mit konventionellen Mitteln, wie z.B. Thionylchlorid oder Phosphoroxychlorid, erhalten werden.

Die Ausgangsverbindungen der Formel VI werden durch Kondensation einer Carboxylsäure einer Verbindung der Formel IX oder eines reaktionsfähigen funktionellen Derivat davon mit einem der Gruppe $R_4$ der Formel I entsprechenden Amin, nämlich einer Verbingung der Formel $R_4$-H, worin $R_4$ die oben angegebene Bedeutung hat und H an dem Stickstoffatom der Aminogruppe $R_4$ gebunden ist, in an sich bekannter Weise erhalten.

Ein reaktionsfähiges funktionelles Derivat einer Carbonsäure einer Verbindung der Formel IX darstellt z.B. ein Halogenacryl, wie Säurechlorid, ein Anhydrid einer solchen Säure, ein gemischtes Anhydrid, z.B. von einem Niederalkylhalogenkarbonat abgeleitetes, wie Ethylchlorformiat, ein reaktionsfähiger Ester, wie Niederalkylester, z.B. Ethyl- oder Methylester oder ein gegebenenfalls substituierter Phenylester, oder ein Amid, z.B. von Imidazol abgeleitet (hergestellt aus N,N-Carbonyldiimidazol).

Die Kondensation eines gegebenenfalls in geschützter Form vorliegenden Amins der Formel III ($R_4$-H) mit einer Verbindung der Formel IX in Form einer freien Carbonsäure wird vorzugsweise in Gegenwart eines Kondensationsmittels, wie Dicyclohexylcarbodiimid oder 1,1-di-Imidazolylcarbonyl (Carbonyldiimidazol) in einem inerten Lösungsmittel, wie Methylenchlorid oder Tetrahydrofuran, bei Temperaturen nahe dem Siedepunkt des Lösungsmittels durchgeführt.

Die Kondensation einer Verbindung der Formel III mit einer Verbindung der Formel IX, welche in Form eines reaktionsfähigen Derivats der Carbonsäure, wie ein Säurehalid, vorzugsweise Säurechlorid, oder ein gemischtes Anhydrid, vorliegt, wird in einem inerten Lösungsmittel, wie Toluol oder Methylenchlorid, vorzugsweise in Gegenwart einer Base, z.B. einer anorganischen Base, wie Kaliumkarbonat, oder in einer organischen Base, wie Triethylamin oder Pyridin, bei Temperaturen von etwa 0° bis 100°, vorzugsweise bei Zimmertemperatur

durchgeführt.

Die erhaltenen erfindungsgemässen Verbindungen der Formel I können in andere erfindungsgemässe Verbindungen der Formel I in an sich bekannter Weise überführt werden.

So können z.B. Verbindungen der Formel I, worin $R_1$ Niederalkoxy bedeutet, in Verbindungen der Formel I, worin $R_1$ Hydroxy bedeutet, in an sich bekannter Weise, z.B. mit einer Mineralsäure, wie Jodwasserstoffsäure, oder vorzugsweise bei Verbindungen, worin $R_1$ Methoxy bedeutet, mit Bor-tribromid in Methylenchlorid oder mit Natrium- oder Lithium-diphenylphosphid in Tetrahydrofuran, überführt werden.

Die oben genannten Reaktionen werden nach an sich bekannten Methoden, in Gegenwart oder Abwesenheit von Verdünnungsmitteln, vorzugsweise in solchen, welche gegenüber den Reagenzien inert sind und diese lösen, Katalysatoren, Kondensationsmitteln oder anderen genannten Mitteln, und/oder in einer inerten Atmosphäre, unter Kühlen, bei Zimmertemperatur oder bei erhöhter Temperatur, vorzugsweise beim Siedepunkt des verwendeten Lösungsmittels, bei normalem oder erhöhtem Druck, durchgeführt.

Die Erfindung betrifft ebenfalls Abänderungen des vorliegenden Verfahrens, ein auf irgendeiner Stufe des Verfahrens erhältliches Zwischenprodukt als Ausgangsmaterial verwendet wird und die verbleibenden Verfahrensschritte durchgeführt werden, oder das Verfahren auf irgeneiner Stufe abgeborchen wird, oder wonach ein Ausgangsmaterial unter den Reaktionsbedingungen gebildet, oder worin ein Ausgangsstoff in Form eines Salzes oder von reinen Isomeren verwendet wird.

Beim Verfahren der vorliegenden Erfindung werden vorzugsweise solche Ausgangsstoffe verwendet, welche zu den eingangs als besonders wertvoll geschilderten Verbindungen führen.

Schliesslich können die Verbindungen der Erfindung entweder in freier Form oder in Form ihrer Salze erhalten werden, wann immer eine Verbindung für die Salzbildung geeignet ist. Eine erhaltene freie Base kann in das entsprechende Säureadditionssalz, vorzugsweise mit solchen Säuren oder Anionenaustauschern, welche pharmazeutisch anwendbare Salze ergeben, überführt werden. Ein erhaltenes Salz kann in die entsprechende freie Base, z.B. mit einer stärkeren Base, wie ein Metall- oder Ammoniumhydroxid oder ein basisches Salz, z.B. mit Alkalimetall-hydroxiden oder -carbonaten oder mit Kationenaustauschern, umgewandelt werden. Die Säureadditionssalze bildet man vorzogsweise mit solchen, vorher beschriebenen, anorganischen oder organischen Säuren, welche pharmazeutisch anwendbare Salze ergeben.

Diese oder andere Salze, z.B. Pikrate, können such in der Reinigung der erhaltenen Basen verwendet werden. Die Basen werden in ihre Salze übergeführt, die Salze abgetrennt und die freien Verbindungen aus Salzen freigesetzt.

Infolge der engen Beziehungen zwischen den neuen Verbindungen in freier Form und in Form ihrer Salze sind im vorausgegangenen und nachfolgend unter freien Verbindungen und Salzen sinn- und zweckgemäss gegebenenfalls auch die entsprechenden Salze bzw. freien Verbindungen zu verstehen.

Die Verbindungen und ihre Salze können auch in der form ihrer Hydrate erhalten werden oder andere, zur Krisallisation verwendete Lösungsmittel, enthalten.

Die vorliegende Erfindung betrifft im weiteren Verbindungen der Formel I und ihre therapeutisch annehmbaren, nichttoxischen Säureadditionssalze zur Verwendung als Medikamente, und insbesondere zur Verwendung bei der Herstellung pharmazeutischer Präparate, im besonderen Präparate, die zur Behandlung von Säugern, insbesondere als gastrointestinale Wirkstoffe, wie gastrointestinale Antisekretionsmittel zur Behandlung von gastrointestinalen Sekretionskrankheiten, wie Diarrhöe, Ulcera, inflammatorischen Darmkrankheiten und Dehydratation, verwendet werden können.

Die vorliegende Erfindung betrifft auch die Verwendung der erfindungsgemässen Verbindungen der Formel I zur Herstellung von pharmazeutischen Präparaten, insbesondere gastrointestinalen Antisekretionspräparaten, vorzugsweise antidiarrhöischen pharmazeutischen Präparaten, die eine hemmende Wirkung auf Calmodulin aufweisen.

Bei den erfindungsgemässen Präparaten handelt es sich um solche zur enteralen, wie oralen oder rektalen, sowie zur parenteralen Verabreichung an Säugern einschiesslich der Menschen. Die erfindungsgemässen pharmazeutischen Präparate enthalten mindestens eine Verbindung der Formel I, oder eines ihrer therapeutischen annehmbaren Salze als Wirkstoffe, gegebenenfalls zusammen mit einem oder mehreren therapeutisch annehmbaren Trägerstoffen.

Die pharmakologisch verwendbaren Verbindungen der vorliegenden Erfindung können zur Herstellung von pharmazeutischen Präparaten verwendet werden, welche eine wirksame Menge der Aktivsubstanz zusammen oder im Gemisch mit Trägerstoffen enthalten, die sich zur enteralen oder parenteralen Verabreichung eignen. Vorzogsweise verwendet man Tabletten oder Gelatinekapseln, welche den Wirkstoff zusammen mit a) Verdünnungsmitteln, z.B. Lactose, Dextrose, Rohrzucker, Mannitol, Sorbitol, Cellulose und/oder Glycin, und b) Schmiermitteln, z.B. Kieselerde, Talk, Stearinsäure oder Salze davon, wie Magnesium- oder Calciumstearat, und/oder Polyäthylenglykol, aufweisen ; Tabletten enthalten c) Bindemittel, z.B. Magnesiumaluminiumsilikat, Stärke-Paste, Gelatine, Traganth, Methylcellulose, Natriumcarboxymethylcellulose und/oder Polyvinylpyrrolidon, und, wenn erwünscht, d) Sprengmittel, z.B. Stärken, Agar, Alginsäure oder ein Salz davon, wie Natriumalginat, und/oder Brausemischungen, oder e) Adsorptionsmittel, Farbstoffe, Geschmackstoffe und Süssmittel. Injizierbare Präparate sind vorzugsweise isotonische wässrige Lösungen oder Suspensionen, und Suppositorien in erster Linie Fettemulsionen oder -suspensionen. Die pharmakologischen Präparate können sterilisiert sein und/oder Hilfsstoffe, z.B. Konservier-, Stabilisier-, Netz- und/oder Emulgiermittel, Löslichkeitsvermittler, Salze zur Reguliereung des osmotischen Drucks und/oder Puffer enthalten. Die vorliegenden pharmazeutischen Präparate, die, wenn erwünscht, weitere pharmakologisch wertvolle Stoffe enthalten können, werden in an sich bekannter Weise, z.B. mittels konventioneller Misch-, Granulier- oder Dragierverfahren, hergestellt und enthalten von etwa 0,1% bis etwa 75%, insbesondere von etwa 1% bis etwa 50%, des Aktivstoffes.

Die pharmakologisch aktiven, erfindungsgemässen Verbindungen können im weiteren zur Herstellung von pharmazeutischen Präparaten verwendet werden, welche eine wirksame Menge der Aktivsubstanz zusammen oder im Gemisch mit Trägerstoffen enthalten, die sich zur transdermalen Applikation eignen. Vorzugsweise verwendet man bei dieser Formulierung Applikationen in Form von Bandagen, die aus einer Rückseite, einem Reservoir enthaltend die Aktivsubstanz, gegebenenfalls mit Trägerstoffen, gegebenenfalls eine steuerbare Membrane für die kontrollierte Abgabe der Aktivsubstanz in die Haut einer bestimmten Zeitperiode, und Hilfsmittel zur Sicherung der Vorrichtung an der Haut bestehen.

Insbesondere betrifft die vorliegende Erfindung Methoden zur Behandlung von Krankheiten bei Säugern, welche durch Hemmung von Calmodulin beeinflusst werden, vorzugsweise bei gastrointestinalen Sekretionskrankheiten, wie Diarrhöe, Ulcera, inflammatorischen Darmkrankheiten und Dehydratation, indem man eine wirksame Menge einer erfindungsgemässen Verbindung der Formel I oder eines ihrer therapeutisch annehmbaren Salzes als Wirkstoff, insbesondere in Form oben beschriebenen pharmazeutischen Präparaten verwendet. Die Dosierung des Wirkstoffs hängt von Warmblüterspezies, deren Körpergewicht, Alter und dem individuellen Zustand sowie der Applikationsweise ab. Einzeldosen für Säuger mit einem Gewicht von ungefähr 50-70 kg können zwischen ungefähr 5 und 100 mg des aktiven Bestandteils enthalten.

Die folgenden Beispiele dienen zur Illustration der Erfindung ; Temperaturen werden in Celsiusgraden angegeben. Wenn nicht anders angegeben, werden alle Verdampfungs-Vorgänge bei vermindertem Druck, vorzugsweise zwischen ungefähr 15 und 100 mm Hg ($\hat{=}$ 2 bis 13.3 kPa), durchgeführt. Die Struktur der Endprodukte, Zwischenprodukte und Ausgangsstoffe wird durch analytische Methoden, wie Mikroanalyse und spektroskopische Charakteristika (z.B. IR, NMR), bestätigt.

Beispiel 1

1,3-Dihydro-1-{1-[(4-methyl-4H,6H-pyrrolo-[1,2-a][4,1]-benzoxazepin-4-yl)-methyl]-4-piperidinyl}-2H-benzimidazol-2-on (1 : 1) Maleat.

Zu einer Suspension von 3,0 g 1,3-Dihydro-1-{1-[(4-methyl-4H,6H-pyrrolo-[1,2-a][4,1]-benzoxazepin-4-yl)-carbonyl]-4-piperidinyl}-2H-benzimidazol-2-on in 120 ml Tetrahydrofuran wird 700 mg Lithium-aluminiumhydrid zugegeben. Das Reaktionsgemisch wird 6 Stunden unter Rückfluss und dann 18 Stunden bei Raumtemperatur gerührt. Das Reaktionsgemisch wird mit 150 ml Ether verdünnt und der Ueberschuss an Lithiumaluminiumhydrid wie unten beschrieben beseitigt. Man filtriert den Feststoff ab. Die organische Phase wird mit 150 ml NaOH Lösung und dann mit 150 ml Salzlauge gewaschen. Die organischen Extrakte werden dann über Magnesiumsulfat getrocknet, filtriert und das Lösungsmittel im Vakuum abgezogen, wobei man 1,3-Dihydro-1-{1-[(4-methyl-4H,6H-pyrrolo[1,2-a][4,1]-benzoxazepin-4-yl)-methyl]-4-piperidinyl}-2H-benzimidazol-2-on, F. 199-201°, Verbindung der Formel II, worin $R_1$ und $R_5$ = H und $R_2$ = $CH_3$ und n = 1 bedeuten, erhält.

Die erhaltene Verbindung wird dann in ihr Maleatsalz überführt, indem man die freie Base und eine molar-äquivalente Menge Maleinsäure in Aceton zusammenfügt. Das 1 : 1 Maleatsalz kristallisiert aus und hat einen Schmelzpunkt von 183-185°.

Das 1 : 1 Fumaratsalz wird in analoger Weise hergestellt und hat einen Schmelzpunkt von 125-127°.

Der Ausgangsstoff wird wie folgt hergestellt :

Ein Gemisch von 500 g Methylantranilat und 438 g 2,5-Dimethoxytetrahydrofuran in 670 ml Eisessigsäure wird 1 1/2 Stunden unter Rückfluss gekocht. Die Essigsäure wird dann im Vakuum abgedampft und der Rückstand destilliert, man erhält 2-(1H-Pyrrol-1-yl)-benzonsäure-methylester mit Kochpunkt von 109°/0.1 mm Hg.

Zu einer Suspension von 117 g Lithiumaluminiumhydrid in 2 l wasserfreiem Ether (unter inerter Atmosphäre) wird 4 Stunden lang tropfenweise eine Lösung von 428 g 2-(1H-Pyrrol-1-yl)-benzonsäure-methylester in 1,5 l Ether zugefügt. Das Reaktionsgemisch wird weitere 4 Stunden unter Rückfluss gekocht, dann auf Zimmertemperatur abgekühlt.

Nach der Kühlung im Eisbad wird 1 Stunde tropfenweise 117 ml Wasser zugefügt um den Lithiumaluminiumhydrid-Ueberschuse aufzulösen, dann wird tropfenweise 117 ml 15% Natriumhydroxid zugegeben und

später wird während 30 Minuten noch 351 ml Wasser zugefügt. Der feste Rückstand wird abfiltriert, die Ether Schicht dann über Magnesiumsulfat getrocknet und das Lösungsmittel im Vakuum entfernt, man erhält 2-(Pyrrol-1-yl)-benzyl-alkohol, welches dann durch Destillation im Vakuum gereinigt wird, K.P. 110-114°/0.1 mm Hg.

Zu einer Lösung von 34,6 g 2-(Pyrrol-1-yl)-benzyl-alkohol in 300 ml wasserfreiem Tetrahydrofuran und 32 ml Tetramethylethylen-diamin (TMEDA) wird 183 ml einer 2,4 molaren Lösung von n-Butyllithium in solcher Menge zugefügt, dass die innere Temperatur der Reaktion unter 30° fällt. Nach dem Abschluss der Zugabe wird das Reaktionsgemisch 3 Stunden bei Raumtemperatur geführt. Das Reaktionsgemisch wird dann unter Verwendung von Trockeneis/Aceton-Bad auf −70° abgekühlt, und 24 ml Ethylpyrivat innerhalb 1/Minute hinzugefügt. Das Reaktionsgemisch wird auf Raumtemperatur erwärmt und über Nacht (18 Stunden) gerührt.

Das Reaktionsgemisch wird dann in ein Eis-Wasser/Ether Gemisch gegossen und die organische Phase abgezogen, über Magnesiumsulfat getrocknet und das Lösungsmittel im Vakuum verdampft, und man erhält 4-Methyl-4H,6H-pyrrolo[1,2-a][4,1]benzoxazepin-4-carbonsäure-ethyl-ester, F. 94-96°, welches aus Ether-Hexan (1 : 1) umkristallisiert werden kann.

Das Gemisch von 41 g davon, 180 ml 3 N Natriumhydroxid und 150 ml Ethanol wird 6 Stunden unter Rückfluss gekocht. Das Ethanol wird dann im Vakuum abgedampft und die wässerige Lösung mit 6 N HCl auf pH 5 gesäuert. Das erhaltene Produkt, 4-Methyl-4H,6H-pyrrolo[1,2-a][4,1]benzoxazepin-4-carbonsäure, F. 182-183°, wird abfiltriert und kann aus wässerigem Ethanol umkristallisiert werden. Zu einer Lösung von 7,5 g davon, in 300 ml Tetrahydrofuran wird 5 g 1,1-Carbonyldiimidazol hinzugefügt und das erhaltene Gemisch 1 Stunde bei Raumtemperatur gerührt. Zu diesem Gemisch wird 5 g 1,3-Dihydro-1-(4-piperidyl)-2H-benzimidazol-2-on zugegeben, und das Reaktionsgemisch 48 Stunden unter Rückfluss gekocht. Nach Abkühlung auf Raumtemperatur wird das Reaktionsgemisch in 150 ml Eiswasser gegossen und in 150 ml Methylenchlorid extrahiert. Der organische Extrakt wird nacheinander mit 150 ml Natriumcarbonatlösung, 150 ml Wasser und 150 ml verdünnter Salzsäure gewaschen, dann über Magnesiumsulfat getrocknet, filtriert, und das Lösungsmittel im Vakuum abgedampft und man erhält 1,3-Dihydro-1-{1-[(4-methyl-4H,6H-pyrrolo[1,2-a][4,1]benzoxazepin-4-yl)-carbonyl]-4-piperidinyl}-2H-benzimidazol-2-on, F. 208-210°.

Beispiel 2

1,3,4,5-Tetrahydro-1-{1-[(4-methyl-4H,6H-pyrrolo[1,2-a][4,1]benzoxazepin-4-yl)-methyl)-piperidinyl}-2H-imidazol-2-on

Zu einer Lösung von 4,5 g 4-Formyl-4-methyl-4H,6H-pyrrolo[1,2-a][4,1]benzoxazepin in 50 ml Methanol wird 3,6 ml 5,5 N methanolischer HCl und 3,4 g 1,3,4,5-Tetrahydro-1-(4-piperidinyl)-2H-imidazol-2-on und nachher 900 mg Natriumcanborhydrid zugegeben. Das Reaktionsgemisch wird 8 Tage bei Raumtemperatur gerührt. 2 ml konz. HCl wird zum Reaktionsgemisch hinzugefügt und das Lösungsmittel im Vakuum verdampft. Der Rückstand wird in 150 ml Wasser gelöst und mit 150 ml 1 : 1 Ethylacetat-Ether Mischung gewaschen. Die wässerige Phase wird abgetrennt, mit Natriumhydroxidlösung zu pH 9 basisch gemacht und mit 2 × 150 ml Ethylacetat extrahiert. Das vereinigte Ethylacetatextrakt wird über wasserfreiem $MgSO_4$ getrocknet, filtriert und Lösungsmittel im Vakuum abgedampft, und man erhält 1,3,4,5-Tetrahydro-1-{1-[(4-methyl-4H,6H-pyrrolo[1,2-a][4,1]benzoxazepin-4-yl)methyl]-4-piperidinyl}-2H-imidazol-2-on, charakterisiert als sein Maleatsalz, F. 197-199°.

Das Ausgangsmaterial wird wie folgt hergestellt :

Eine Lösung von 7,0 g 4-Methyl-4H,6H-pyrrolo[1,2-a][4,1]benzoxazepin-4-carbonsäure-methylester (hergestellt wie im Beispiel 1) in 200 ml Methylenchlorid wird zu −65° abgekühlt und 65 ml 1,0 m Diisobutylaluminiumhydrid in Methylenchlorid tropfenweise innerhalb von 30 Minuten hinzugefügt. Nach Beendigung der Zugabe wird das Reaktionsgemisch 30 Minuten bei −60° gerührt nachfolgend mit Methanol abgeschreckt. Wasser wird dann zugegeben und das Kühlbad entfernt. Das Reaktionsgemisch wird 15 Minuter gerührt um Raumtemperatur zu erreichen. Man filtriert den Feststoff ab, und die Methylenchloridlösung wird dann über wasserfreiem $MgSO_4$ getrocknet und das Lösungsmittel im Vakuum abgedampft, man erhält einen öligen Rückstand, welcher nach Umkristallisation aus Ether 4-Formyl-4-methyl-4H,6H-pyrrolo[1,2-a)[4,1]benzoxazepin ergibt, F. 83-85°.

Beispiel 3

Die folgenden Verbindungen werden im wesentlichen gemäss den Verfahren der vorherigen Beispielen hergestellt :

a) 8-[(4-Methyl-4H, 6H-pyrrolo[1,2-a] [4,1]benzoxazepin-4-yl)methyl]-1-phenyl-1,3,8-triazaspiro[4,5]-decan-4-on ;

b) 1-[(4-Methyl-4H, 6H,pyrrolo[1,2-a] [4,1]benzoxazepin-4-yl)methyl]-4-hydroxy-4-phenylpiperdin 1 : 1 Maleat, F. 175-177° ;

c) 1-[(4-Methyl-4H, 6H-pyrrolo[1,2-a] [4,1]benzoxazepin-4-yl)methyl]-4-hydroxy-4-(p-chlorophenyl)-piperidin ;

d) 1-[(4-Methyl-4H, 6H-pyrrolo[1,2-a] [4,1]benzoxazepin-4-yl)methyl]-4-phenylpiperazin-dihydrochlorid, F. 155-157° ;

e) 1-[(4-Methyl-4H, 6H-pyrrolo[1,2-a] [4,1]benzoxazepin-4-yl)methyl]-4-phenylpiperidin 1 : 1 Maleat, F. 129-131° ;

f) 1-[(4-Methyl-4H, 6H-pyrrolo[1,2-a] [4,1]benzoxazepin-4-yl)methyl]-4-methylpiperazin 1 : 1 Malest, F. 128-130° ;

g) 1,3-Dihydro-1-{1-[(8-chloro-4-methyl-4H, 6H-pyrrolo[1,2-a] [4,1]benzoxazepin-4-yl)-methyl]-4-piperidinyl}-2H-benzimidazol-2-on.

Beispiel 4

A) Herstellung von 10.000 Tabletten mit einem Gehalt von 10 mg der aktiven Substanz :

| Bestandteile: | |
|---|---|
| 1,3-Dihydro-1-{1-[(4-methyl-4H,6H-pyrrolo-[1,2-a][4,1]-benzoxazepin-4-yl)-methyl]-4-piperidinyl}-2H-benzimidazol-2-on 1:1 Maleat | 100,00 g |
| Milchzucker | 2.400,00 g |
| Maisstärke | 125,00 g |
| Polyäthylenglykol 6000 | 150,00 g |
| Magnesiumstearat | 40,00 g |
| gereinigtes Wasser | q.s. |

Verfahren :

Sämtliche pulverigen Bestandteile werden mit einem Sieb von 0,6 mm Maschenweite gesiebt. Dann wird der Wirkstoff mit Milchzucker, Magnesiumstearat und mit der Hälfte der Stärke in einem geeigneten Mischer vermischt. Die andere Hälfte der Stärke wird in 65 ml Wasser suspendiert und die Suspension zur siedenden Lösung von Polyäthylenglykol in 260 ml Wasser gegeben. Die erhaltene Paste wird zu den Pulvern gegeben und gegebenenfalls unter Zugabe einer weiteren Wassermenge granuliert. Das Granulat wird über Nacht bei 35° getrocknet, durch ein Sieb von 1,2 mm Maschenweite getrieben und zu Tabletten gepresst.

B) Herstellung von 10.000 Kapseln mit einem Gehalt von 10 mg der aktiven Substanz :

| Bestandteile: | |
|---|---|
| 1,3-Dihydro-1-{1-[(4-methyl-4H,6H-pyrrolo-[1,2-a][4,1]-benzoxazepin-4-yl)-methyl]-4-piperidinyl}-2H-benzimidazol-2-on 1:1 Maleat | 10,00 g |
| Milchzucker | 207,00 g |
| modif. Stärke | 80,00 g |
| Magnesiumstearat | 3,00 g |

Verfahren :

Sämtliche pulverigen Bestandteile werden mit einem Sieb von 0,6 mm Maschenweite gesiebt. Dann wird der Wirkstoff zuerst mit Magnesiumstearat und darauffolgend mit Milchzucker und Stärke in einem geeigneten Mischer homogenisiert. Kapseln Nr. 2 werden mit je 300 mg des Gemisches mit einer Füllmaschine gefüllt.

In analoger Weise oder wie weiter vorne beschrieben werden Tabletten oder Kapseln mit Gehalt von etwa 5-100 mg der anderen erfindungsgemässen Verbindungen, z.B. solchen, die in den weiteren Beispielen hier

illustriert sind, hergestellt.

## Ansprüche

**Patentansprüche für die benannten Vertragsstaaten : BE, CH, DE, FR, GB, IT, LI, LU, NL, SE**

1. Verbindungen der allgemeinen Formel I

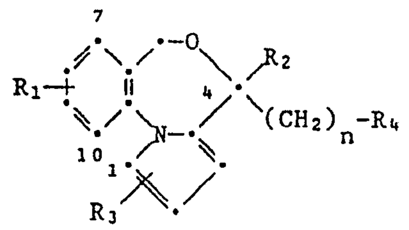

(I),

worin n für eine ganze Zahl 1 oder 2, steht ; $R_1$ Wasserstoff, Niederalkyl, Halogen, Trifluormethyl, Hydroxy oder Niederalkoxy bedeutet ; $R_2$ Wasserstoff oder Niederalkyl ist ; $R_3$ Wasserstoff, Niederalkyl oder Halogen bedeutet ; $R_4$ N-Niederalkyl piperazino oder unsubstituiertes oder durch Halogen oder Niederalkyl im Phenylrest substituiertes N-Phenylpiperazino, Piperidino oder substituiertes Piperidino ausgewählt aus

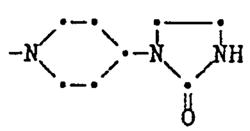

,

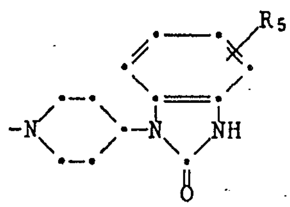

,

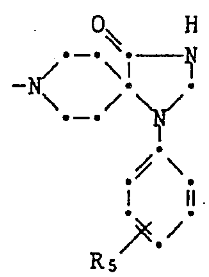

,

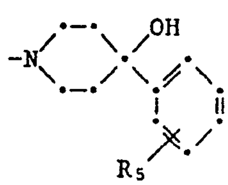

und

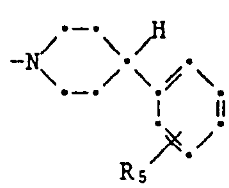

,

worin $R_6$ Wasserstoff, Halogen oder Niederalkyl bedeutet, darstellt ; und Salze davon, wobei die mit "nieder" bezeichneten Reste höchstens 7 Kohlenstoffatome aufweisen.

2. Verbindungen der Formel II

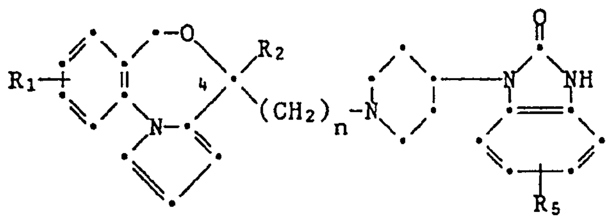

(II),

worin $R_1$ Wasserstoff, Niederalkyl, Halogen, Trifluormethyl, Hydroxy oder Niederalkoxy bedeutet ; $R_2$ Wasserstoff oder Niederalkyl ; n eine ganze Zahl 1 oder 2 darstellt ; und $R_5$ Wasserstoff, Halogen oder Niederalkyl

bedeutet ; und pharmazeutisch annehmbare Salze davon, wobei die mit "nieder" bezeichneten Reste höchstens 7 Kohlenstoffatome aufweisen.

3. Verbindungen der in Anspruch 2 gezeigten Formel II, worin n für eine ganze Zahl 1 oder 2 steht ; $R_2$ geradkettiges Niederalkyl mit 1 bis 4 Kohlenstoffatomen bedeutet ; $R_1$ und $R_5$ Wasserstoff oder Halogen darstellen, und pharmazeutisch annehmbare Salze davon.

4. Verbindungen der im Anspruch 2 gezeigten Formel II, worin $R_1$ und $R_5$ Wasserstoff bedeuten ; $R_2$ Methyl darstellt ; und n 1 bedeutet ; und pharmazeutisch annehmbare Salze davon.

5. 1,3-Dihydro-1-{1-[(4-methyl-4H, 6H-pyrrolo[1,2-a] [4,1]benzoxazepin-4-yl)-methyl]-4-piperidinyl}-2H-benzimidazol-2-on und seine pharmazeutisch annehmbaren Salze.

6. Pharmazeutische Präparate enthaltend Verbindungen der Ansprüche 1-5, zusammen mit einem pharmazeutischen Trägermaterial.

7. Die in den Ansprüche 1-5 genannten Verbindungen zur Anwendung in einem Verfahren zur therapeutischen Behandlung des menschlichen und tierischen Körpers.

8. Die in den Ansprüchen 1-5 genannten Verbindungen zur Verwendung als Antidiarrhoikum.

9. Die in den Ansprüchen 1-5 genannten Verbindungen zur Verwendung als Gastrointestinalsekretions-Hemmer.

10. Verwendung der in den Ansprüchen 1-5 genannten Verbindungen zur Herstellung von pharmazeutischen Präparaten.

11. Verfahren zur Herstellung von den im Anspruch 1 genannten Verbindungen der Formel I und Salzen davon, dadurch gekennzeichnet, dass man

a) ein Amin der Formel III

$$R_4\text{-H} \quad \text{(III)}$$

worin $R_4$ die in Anspruch 1 angegebene Bedeutung hat und H an dem Stickstoffatom der Gruppe $R_4$ gebunden ist, mit einer Verbindung der Formel IV

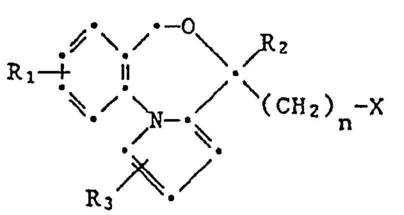

(IV),

worin n, $R_1$, $R_2$ und $R_3$ die unter Formel I angegebene Bedeutung haben und X eine reaktionsfähige veresterte Hydroxygruppe bedeutet ; oder mit einer Verbindung der Formel V

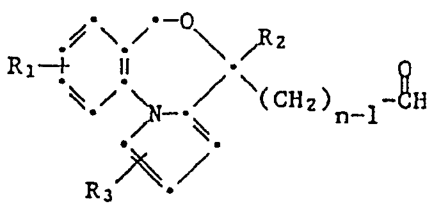

(V),

worin n, $R_1$, $R_2$ und $R_3$ die in Anspruch 1 unter Formel I angegebene Bedeutung haben, unter Bedingungen einer reduktiven Aminierung ; kondensiert ; oder

b) eine Verbindung der Formel VI

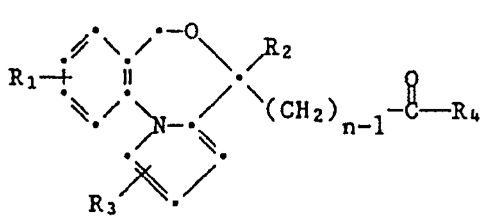

(VI),

worin $R_1$, $R_2$, $R_3$, $R_4$ und n die unter Formel I im Anspruch 1 angegebene Bedeutung haben, reduziert und, wenn erwünscht, in allen diesen Verfahren eine störende reaktionsfähige Gruppe vorübergehend schützt, und die erhaltene Verbindung der Formel I isoliert; und, wenn erwünscht, eine erhaltene Verbindung der Formel I in eine andere erfindungsgemässe Verbindung der Formel I umwandelt, und/oder, wenn erwünscht, eine erhaltene Verbindung der Formel I in ihr Salz oder ein erhaltenes Salz in die freie Verbindung oder in ein anderes Salz überführt, und/oder wenn erwünscht, ein erhaltenes Gemisch von Isomeren oder Razematen in die einzelnen Isomeren oder Razemate auftrennt, und/oder wenn erwünscht, erhaltene Razemate in die optischen Antipoden trennt.

12. Die nach dem Verfahren des Anspruchs 11 erhältlichen Verbindungen.

13. Verbindungen der allgemeinen Formel IV

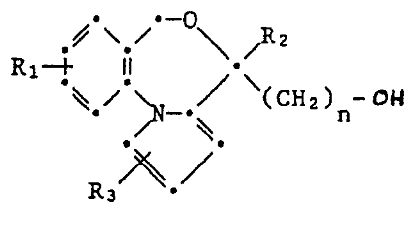

(IV),

worin n für eine ganze Zahl 1 oder 2, steht; $R_1$ Wasserstoff, Niederalkyl, Halogen, Trifluormethyl, Hydroxy oder Niederalkoxy bedeutet; $R_2$ Wasserstoff oder Niederalkyl bedeutet; $R_3$ Wasserstoff, Niederalkyl oder Halogen ist; wobei die mit "nieder" bezeichneten Reste höchstens 7 Kohlenstoffatome aufweisen.

14. Verbindungen der allgemeinen Formel V

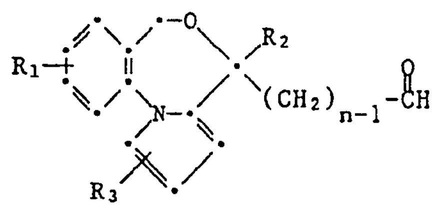

(V),

worin n für eine ganze Zahl 1 oder 2, steht; $R_1$ Wasserstoff, Niederalkyl, Halogen, Trifluormethyl, Hydroxy oder Niederalkoxy bedeutet; $R_2$ Wasserstoff oder Niederalkyl ist; und $R_3$ Wasserstoff, Niederalkyl oder Halogen ist, wobei die mit "nieder" bezeichneten Reste höchstens 7 Kohlenstoffatome aufweisen.

15. Verbindungen der allgemeinen Formel IX

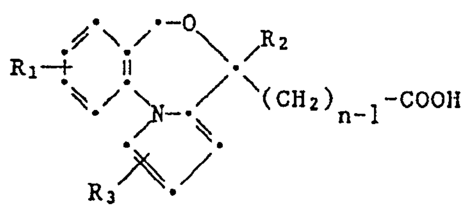

(IX),

worin n für eine ganze Zahl 1 oder 2, steht; $R_1$ Wasserstoff, Niederalkyl, Halogen, Trifluormethyl, Hydroxy oder Niederalkoxy bedeutet; $R_2$ Wasserstoff oder Niederalkyl ist; und $R_3$ Wasserstoff, Niederalkyl oder Halogen ist, wobei die mit "nieder" bezeichneten Reste höchstens 7 Kohlenstoffatome aufweisen.

16. Verbindungen der allgemeinen Formel VI

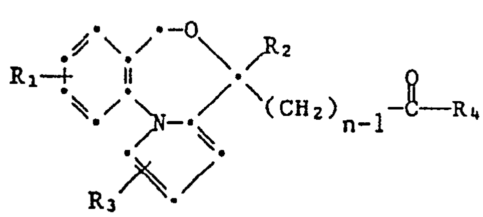

(VI),

worin n für eine ganze Zahl 1 oder 2, steht ; $R_1$ Wasserstoff, Niederalkyl, Halogen, Trifluormethyl, Hydroxy oder Niederalkoxy bedeutet ; $R_2$ Wasserstoff oder Niederalkyl ist ; $R_3$ Wasserstoff, Niederalkyl oder Halogen darstellt ; $R_4$ N-Niederalkyl-piperazino oder unsubstituiertes oder durch Halogen oder Niederalkyl im Phenylrest substituiertes N-Phenyl-piperazino, Piperidino oder substituiertes Piperidino ausgewählt aus

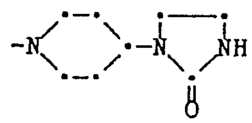 , 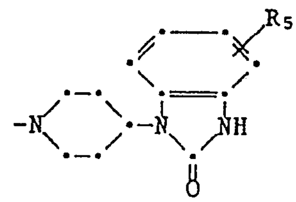 , 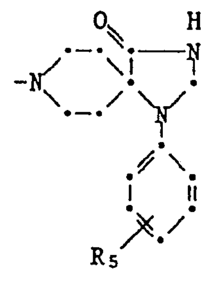 ,

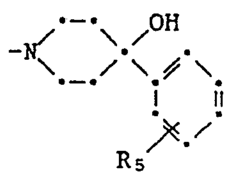 und 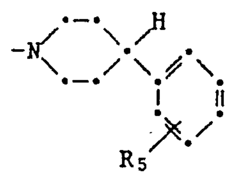

worin $R_5$ Wasserstoff, Halogen oder Niederalkyl bedeutet, darstellt, wobei die mit "nieder" bezeichneten Reste höchstens 7 Kohlenstoffatome aufweisen.

**Patentansprüche für die benannten Vertragsstaaten : AT, ES, GR**

1. Verfahren zur Herstellung von Verbindungen der allgemeinen Formel I

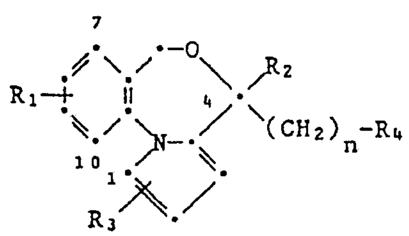 (I),

worin n für eine ganze Zahl 1 oder 2, steht ; $R_1$ Wasserstoff, Niederalkyl, Halogen, Trifluormethyl, Hydroxy oder Niederalkoxy bedeutet ; $R_2$ Wasserstoff oder Niederalkyl ist ; $R_3$ Wasserstoff, Niederalkyl oder Halogen bedeutet ; $R_4$ N-Niederalkyl-piperazino oder unsubstituiertes oder im Phenylrest durch Halogen oder Niederalkyl substituiertes N-Phenylpiperazino, Piperidino oder substituiertes Piperidino ausgewählt aus

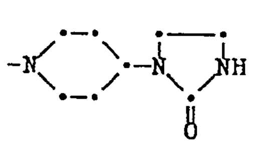 , 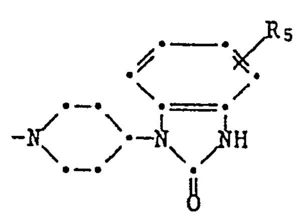 , 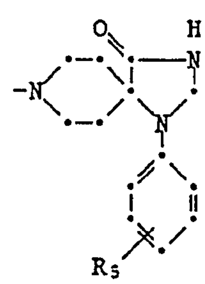 ,

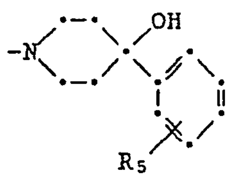 und 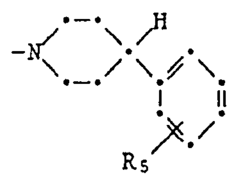 ,

worin $R_5$ Wasserstoff, Halogen oder Niederalkyl bedeutet, darstellt ; und Salzen davon, wobei die mit "nieder" bezeichneten Reste höchstens 7 Kohlenstoffatome aufweisen dadurch gekennzeichnet, dass man

a) ein Amin der Formel III

$R_4$-H (III),

worin $R_4$ die oben angegebene Bedeutung hat und H an dem Stickstoffatom der Gruppe $R_4$ gebunden ist, mit einer Verbindung der Formel IV

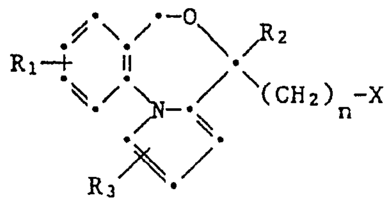 (IV),

worin n, $R_1$, $R_2$ und $R_3$ die unter Formel I angegebene Bedeutung haben und X eine reaktionsfähige veresterte Hydroxygruppe bedeutet ; oder mit einer Verbindung der Formel V

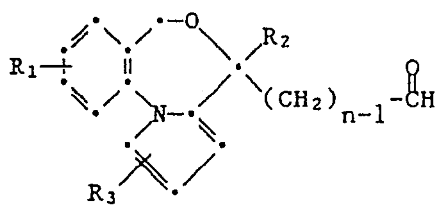 (V),

worin n, $R_1$, $R_2$ und $R_3$ die unter Formel I angegebene Bedeutung haben, unter Bedingungen einer reduktiven Aminierung ; kondensiert ; oder

b) eine Verbindung der Formel VI

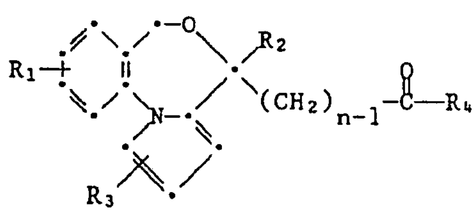 (VI),

worin $R_1$, $R_2$, $R_3$, $R_4$ und n die unter Formel I angegebene Bedeutung haben, reduziert und, wenn erwünscht, in allen diesen Verfahren eine störende reaktionsfähige Gruppe vorübergehend schützt, und die erhaltene Verbindung der Formel I isoliert ; und, wenn erwünscht, eine erhaltene Verbindung der Formel I in eine andere erfindungsgemässe Verbindung der Formel I umwandelt, und/oder, wenn erwünscht, eine erhaltene Verbindung der Formel I in ihr Salz oder ein erhaltenes Salz in die freie Verbindung oder in ein anderes Salz überführt, und/oder wenn erwünscht, ein erhaltenes Gemisch von Isomeren oder Razematen in die einzelnen Isomeren oder Razemate auftrennt, und/oder wenn erwünscht, erhaltene Razemate in die optischen Antipoden trennt.

2. Verfahren nach Anspruch 1 zur Herstellung von Verbindungen der Formel II

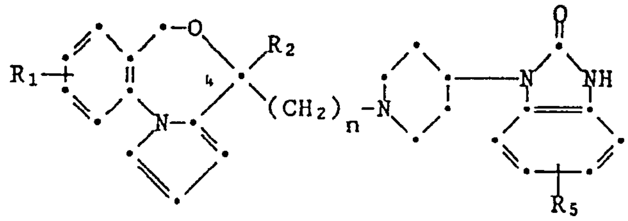

(II),

worin $R_1$ Wasserstoff, Niederalkyl, Halogen, Trifluormethyl, Hydroxy oder Niederalkoxy bedeutet ; $R_2$ Wasserstoff oder Niederalkyl ; n eine ganze Zahl von 1 oder 2 darstellt ; und $R_5$ Wasserstoff, Halogen oder Niederalkyl bedeutet ; und pharmazeutisch annehmbaren Salzen davon, wobei die mit "nieder" bezeichneten Reste höchstens 7 Kohlenstoffatome aufweisen.

3. Verfahren nach Anspruch 1 zur Herstellung von Verbindungen der in Anspruch 2 gezeigten Formel II, worin n für eine ganze Zahl 1 oder 2 steht ; $R_2$ geradkettiges Niederalkyl mit 1 bis 4 Kohlenstoffatomen bedeutet; $R_1$ und $R_5$ Wasserstoff oder Halogen darstellen, und pharmazeutisch annehmbaren Salzen davon.

4. Verfahren nach Anspruch 1 zur Herstellung von Verbindungen der im Anspruch 2 gezeigten Formel II, worin $R_1$ und $R_5$ Wasserstoff bedeuten ; $R_2$ Methyl darstellt ; und n 1 bedeutet ; und pharmazeutisch annehmbaren Salzen davon.

5. Verfahren nach Anspruch 1 zur Herstellung von 1,3-Dihydro-1-{1-[(4-methyl-4H,6H-pyrrolo[1,2-a][4,1]benzoxazepin-4-yl)-methyl]-4-piperidin-yl}-2H-benzimidazol-2-on und seine pharmazeutisch annehmbaren Salzen.

6. Verfahren zur Herstellung eines pharmazeutischen Präparates, gekennzeichnet durch die Verarbeitung eines erfindungsgemässen Wirkstoffes nach einem der Ansprüche 1-5 mit einem pharmazeutischen Trägermaterial.

7. Verwendung von Verbindungen der allgemeinen Formel IV

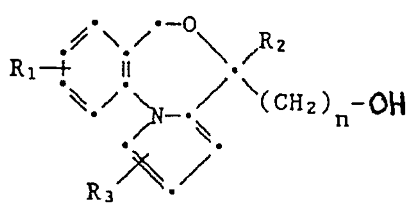

(IV),

worin n für eine ganze Zahl 1 oder 2, steht ; $R_1$ Wasserstoff, Niederalkyl, Halogen, Trifluormethyl, Hydroxy oder Niederalkoxy bedeutet ; $R_2$ Wasserstoff oder Niederalkyl bedeutet ; $R_3$ Wasserstoff, Niederalkyl oder Halogen wobei die mit "nieder" bezeichneten Reste höchstens 7 Kohlenstoffatome aufweisen zur Herstellung von Verbindungen der allgemeinen Formel I gemäss Anspruch 1.

8. Verwendung von Verbindungen der allgemeinen Formel V

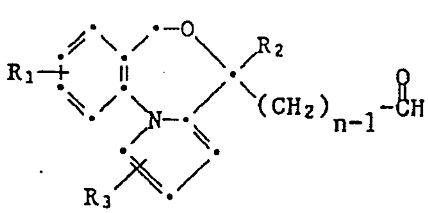

(V),

worin n für eine ganze Zahl 1 oder 2, steht ; $R_1$ Wasserstoff, Niederalkyl, Halogen, Trifluormethyl, Hydroxy oder Niederalkoxy bedeutet ; $R_2$ Wasserstoff oder Niederalkyl ist ; und $R_3$ Wasserstoff, Niederalkyl oder Halogen ist, wobei die mit "nieder" bezeichneten Reste höchstens 7 Kohlenstoffatome aufweisen zur Herstellung von Verbindungen der allgemeinen Formel I gemäss Anspruch 1.

9. Verwendung von Verbindungen der allgemeinen Formel IX

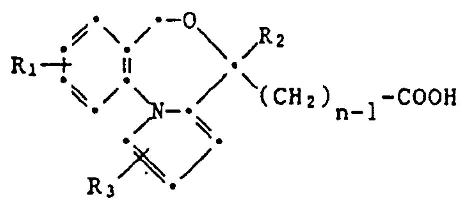

(IX),

worin n für eine ganze Zahl 1 oder 2, steht ; $R_1$ Wasserstoff, Niederalkyl, Halogen, Trifluormethyl, Hydroxy oder Niederalkoxy bedeutet ; $R_2$ Wasserstoff oder Niederalkyl ist ; und $R_3$ Wasserstoff, Niederalkyl oder Halogen ist, wobei die mit "nieder" bezeichneten Reste höchstens 7 Kohlenstoffatome aufweisen zur Herstellung von Verbindungen der allgemeinen Formel I gemäss Anspruch 1.

10. Verwendung von Verbindungen der allgemeinen Formel VI

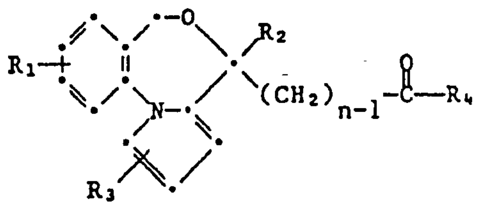

(VI),

worin n für eine ganze Zahl 1 oder 2 Steht ; $R_1$ Wasserstoff, Niederalkyl, Halogen, Trifluormethyl, Hydroxy oder Niederalkoxy bedeutet ; $R_2$ Wasserstoff oder Niederalkyl ist ; $R_3$ Wasserstoff, Niederalkyl oder Halogen darstellt ; $R_4$ N-Niederalkyl-piperazino oder unsubstituiertes oder durch Halogen oder Niederalkyl im Phenylrest substituiertes N-Phenylpiperazino, Piperidino oder substituiertes Piperidino ausgewählt aus

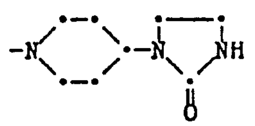 , 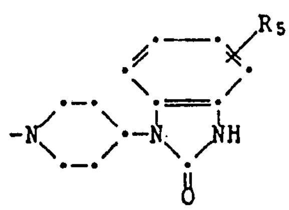 , 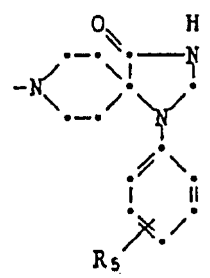 ,

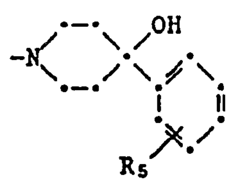 und 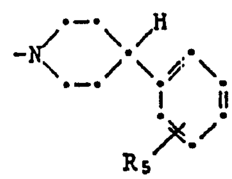 ,

worin $R_5$ Wasserstoff, Halogen oder Niederalkyl bedeutet, darstellt, wobei die mit "nieder" bezeichneten Reste höchstens 7 Kohlenstoffatome aufweisen zur Herstellung von Verbindungen der Formel I gemäss Anspruch 1.

**Claims**

**Claims for the Contracting States : BE, CH, DE, FR, GB, IT, LI, LU, NL, SE**

1. Compounds of the general formula I

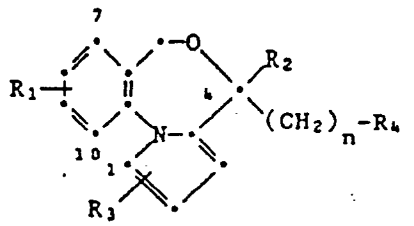

(I)

wherein n represents the integer 1 or 2 ; $R_1$ represents hydrogen, lower alkyl, halogen, trifluoromethyl, hydroxy or lower alkoxy ; $R_2$ represents hydrogen or lower alkyl ; $R_3$ represents hydrogen, lower alkyl or halogen ; $R_4$ represents N-lower alkylpiperazino, N-phenylpiperazino that is unsubstituted or substituted in the phenyl moiety by halogen or by lower alkyl, or piperidino or substituted piperidino selected from

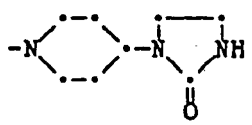

,

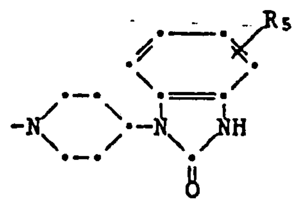

,

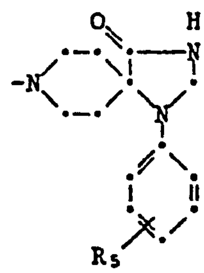

,

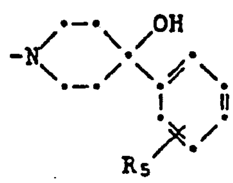

and

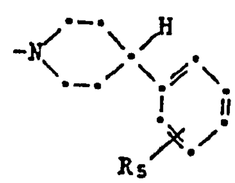

,

in which $R_5$ represents hydrogen, halogen or lower alkyl ; and salts thereof, the radicals designated "lower" having a maximum of 7 carbon atoms

2. Compounds of formula II

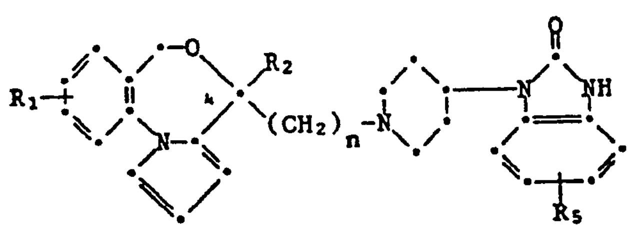

(II)

wherein $R_1$ represents hydrogen, lower alkyl, halogen, trifluoromethyl, hydroxy or lower alkoxy ; $R_2$ represents hydrogen or lower alkyl ; n represents the integer 1 or 2 ; and $R_5$ represents hydrogen, halogen or lower alkyl; and pharmaceutically acceptable salts thereof, the radicals designated "lower" having a maximum of 7 carbon atoms.

3. Compounds of formula II shown in claim 2, wherein n represents the integer 1 or 2 ; $R_2$ represents straight-chain lower alkyl having from 1 to 4 carbon atoms ; $R_1$ and $R_5$ represent hydrogen or halogen, and pharmaceutically acceptable salts thereof.

4. Compounds of formula II shown in claim 2, wherein $R_1$ and $R_5$ represent hydrogen ; $R_2$ represents methyl; and n represents 1 ; and pharmaceutically acceptable salts thereof.

5. 1,3-Dihydro-1-{1-[(4-methyl-4H, 6H-pyrrolo[1,2-a] [4,1]benzoxazepin-4-yl)-methyl]-4-piperidinyl}-2H-benzimidazo1-2-one and pharmaceutically acceptable salts thereof.

6. Pharmaceutical compositions containing compounds of claims 1 to 5 in combination with a pharmaceutical carrier.

7. The compounds mentioned in claims 1 to 5 for use in a method for the therapeutic treatment of the human or animal body.

8. The compounds mentioned in claims 1 to 5 for use as antidiarrhoeal agents.

9. The compounds mentioned in claims 1 to 5 for use as gastrointestinal secretion inhibitors.

10. The use of the compounds mentioned in claims 1 to 5 for the manufacture of pharmaceutical compositions.

11. A process for the preparation of the compounds of formula I mentioned in claim 1, and salts thereof, comprising :

a) condensing an amine of formula III

$R_4$-H (III),

wherein $R_4$ is as defined in claim 1 and H is attached to the nitrogen atom of the group $R_4$, with a compound of formula IV

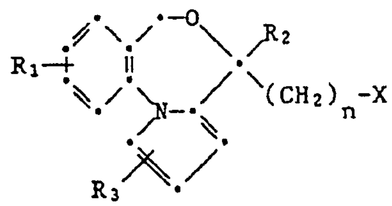

(IV),

wherein n, $R_1$, $R_2$ and $R_3$ are as defined for formula I and X represents reactive esterified hydroxy ; or with a compound of formula V

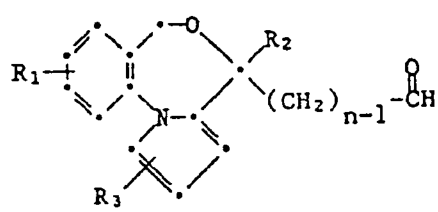

(V),

wherein n, $R_1$, $R_2$ and $R_3$ are as defined for formula I in claim 1, unter conditions of reductive amination ; or

b) reducing a compound of formula VI

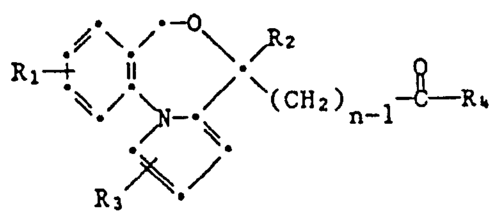

(VI),

wherein $R_1$, $R_2$, $R_3$, $R_4$ and n are as defined for formula I in claim 1 ; and, if desired, in all of these processes temporarily protecting an interfering reactive group, and isolating the resulting compound of formula I ; and, if desired, converting a resulting compound of formula I into another compound of formula I of the invention and/or, if desired, converting a resulting compound of formula I into a salt or a resulting salt into the free compound or into another salt and/or, if desired, separating a resulting mixture of isomers or racemates into the individual isomers or racemates and/or, if desired, resolving racemates obtained into the optical antipodes.

12. The compounds obtainable in accordance with the process of claim 11.

13. Compounds of the general formula IV

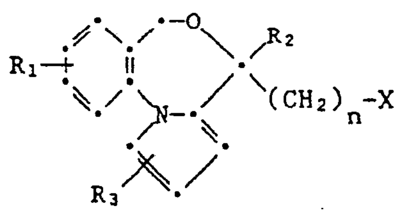

(IV)

wherein n represents the integer 1 or 2 ; $R_1$ represents hydrogen, lower alkyl, halogen, trifluoromethyl, hydroxy or lower alkoxy ; $R_2$ represents hydrogen or lower alkyl ; $R_3$ represents hydrogen, lower alkyl or halogen ; and X represents hydroxy, the radicals designated "lower" having a maximum of 7 carbon atoms.

14. Compounds of the general formula V

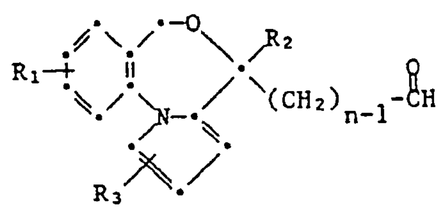

(V)

wherein n represents the integer 1 or 2 ; $R_1$ represents hydrogen, lower alkyl, halogen, trifluoromethyl, hydroxy or lower alkoxy ; $R_2$ represents hydrogen or lower alkyl ; and $R_3$ represents hydrogen, lower alkyl or halogen, the radicals designated "lower" having a maximum of 7 carbon atoms.

15. Compounds of the general formula IX

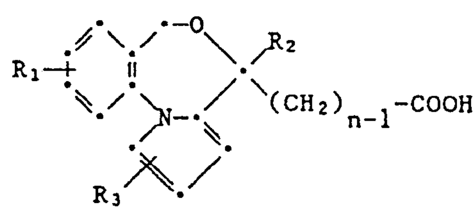

(IX)

wherein n represents the integer 1 or 2 ; $R_1$ represents hydrogen, lower alkyl, halogen, trifluoromethyl, hydroxy or lower alkoxy ; $R_2$ represents hydrogen or lower alkyl ; and $R_3$ represents hydrogen, lower alkyl or halogen, the radicals designated "lower" having a maximum of 7 carbon atoms.

16. Compounds of the general formula VI

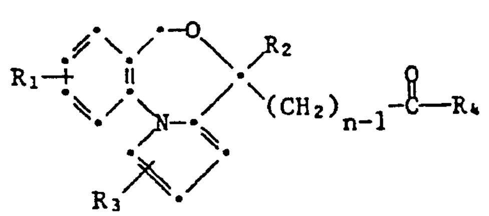

(VI)

wherein n represents the integer 1 or 2 ; $R_1$ represents hydrogen, lower alkyl, halogen, trifluoromethyl, hydroxy or lower alkoxy ; $R_2$ represents hydrogen or lower alkyl ; $R_3$ represents hydrogen, lower alkyl or halogen ; $R_4$ represents N-lower alkylpiperazino, N-phenylpiperazino that is unsubstituted or substituted in the phenyl moiety by halogen or by lower alkyl, or piperidino or substituted piperidino selected from

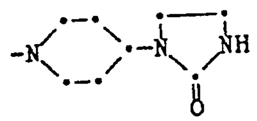 , 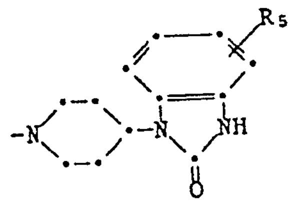 , 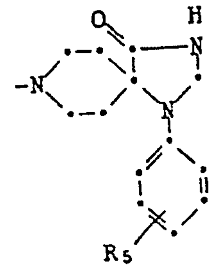 ,

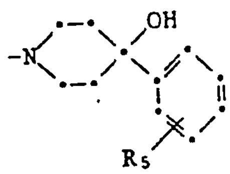 and 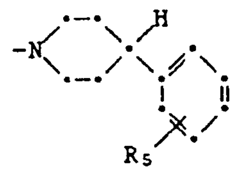

in which $R_5$ represents hydrogen, halogen or lower alkyl, the radicals designated "lower" having a maximum of 7 carbon atoms.

**Claims for the Contracting States : AT, ES, GR**

1. A process for the preparation of compounds of the general formula I

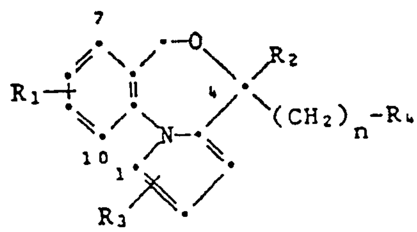

(I)

wherein n represents the integer 1 or 2 ; $R_1$ represents hydrogen, lower alkyl, halogen, trifluoromethyl, hydroxy or lower alkoxy ; $R_2$ represents hydrogen or lower alkyl ; $R_3$ represents hydrogen, lower alkyl or halogen ; $R_4$ represents N-lower alkylpiperazino, N-phenylpiperazino that is unsubstituted or substituted in the phenyl moiety by halogen or by lower alkyl, or piperidino or substituted piperidino selected from

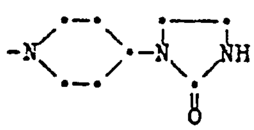 , 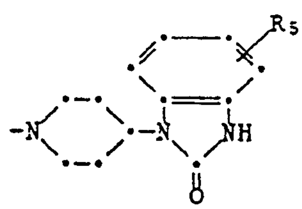 , 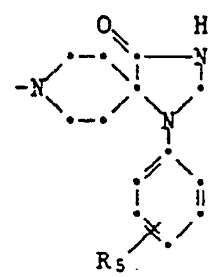 ,

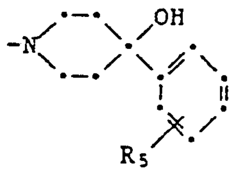

and

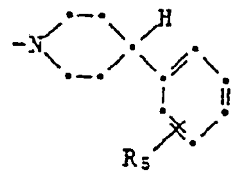

,

in which $R_5$ represents hydrogen, halogen or lower alkyl ; and salts thereof, the radicals designated "lower" having a maximum of 7 carbon atoms, comprising :

a) condensing an amine of formula III

$$R_4\text{-H} \quad \text{(III)},$$

wherein $R_4$ is as defined above and H is attached to the nitrogen atom of the group $R_4$, with a compound of formula IV

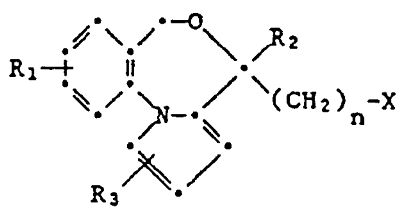

(IV),

wherein n, $R_1$, $R_2$ and $R_3$ are as defined for formula I and X represents reactive esterified hydroxy ; or with a compound of formula V

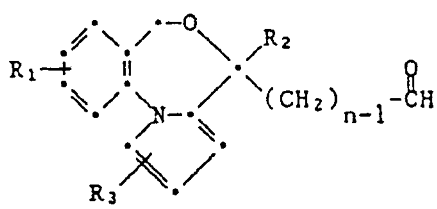

(V),

wherein n, $R_1$, $R_2$ and $R_3$ are as defined for formula I, under conditions of reductive amination ; or

b) reducing a compound of formula VI

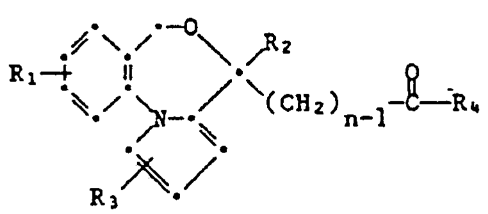

(VI),

wherein $R_1$, $R_2$, $R_3$, $R_4$ and n are as defined for formula I, and, if desired, in all of these processes temporarily protecting an interfering reactive group, and isolating the resulting compound of formula I ; and, if desired, converting a resulting compound of formula I into another compound of formula I of the invention and/or, if desired, converting a resulting compound of formula I into a salt or a resulting salt into the free compound or into another salt and/or, if desired, separating a resulting mixture of isomers or racemates into the individual isomers or racemates and/or, if desired, resolving racemates obtained into the optical antipodes.

2. A process according to claim 1 for the preparation of compounds of formula II

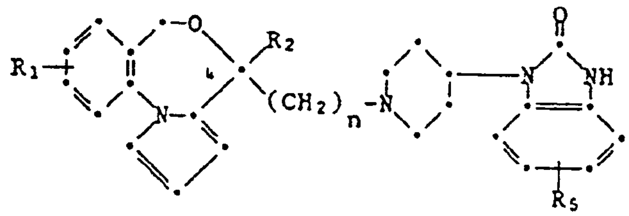

(II)

wherein $R_1$ represents hydrogen, lower alkyl, halogen, trifluoromethyl, hydroxy or lower alkoxy ; $R_2$ represents hydrogen or lower alkyl ; n represents the integer 1 or 2 ; and $R_5$ represents hydrogen, halogen or lower alkyl; and pharmaceutically acceptable salts thereof, the radicals designated "lower" having a maximum of 7 carbon atoms.

3. A process according to claim 1 for the preparation of compounds of formula II shown in claim 2, wherein n represents the integer 1 or 2 ; $R_2$ represents straight-chain lower alkyl having from 1 to 4 carbon atoms ; $R_1$ and $R_5$ represent hydrogen or halogen, and pharmaceutically acceptable salts thereof.

4. A process according to claim 1 for the preparation of compounds of formula II shown in claim 2, wherein $R_1$ and $R_5$ represent hydrogen ; $R_2$ represents methyl ; and n represents 1 ; and pharmaceutically acceptable salts thereof.

5. A process according to claim 1 for the preparation of 1,3-dihydro-1-{1-[(4-methyl-4H,6H-pyrrolo[1,2-a][4,1]benzoxazepin-4-yl)-methyl]-4-piperidinyl}-2H-benzimidazol-2-one and pharmaceutically acceptable salts thereof.

6. A process for the manufacture of a pharmaceutical composition, which comprises processing an active ingredient of the invention according to any one of claims 1 to 5 with a pharmaceutical carrier.

7. The use of compounds of the general formula IV

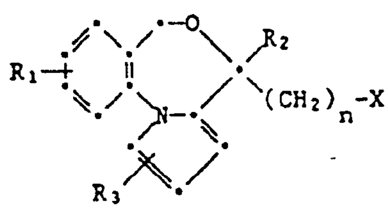

(IV)

wherein n represents the integer 1 or 2 ; $R_1$ represents hydrogen, lower alkyl, halogen, trifluoromethyl, hydroxy or lower alkoxy ; $R_2$ represents hydrogen or lower alkyl ; $R_3$ represents hydrogen, lower alkyl or halogen ; and X represents hydroxy, the radicals designated "lower" having a maximum of 7 carbon atoms, for the preparation of compounds of the general formula I according to claim 1.

8. The use of compounds of the general formula V

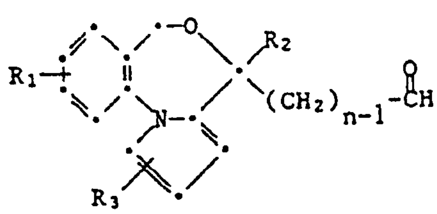

(V)

wherein n represents the integer 1 or 2 ; $R_1$ represents hydrogen, lower alkyl, halogen, trifluoromethyl, hydroxy or lower alkoxy ; $R_2$ represents hydrogen or lower alkyl ; and $R_3$ represents hydrogen, lower alkyl or halogen, the radicals designated "lower" having a maximum of 7 carbon atoms, for the preparation of compounds of the general formula I according to claim 1.

9. The use of compounds of the general formula IX

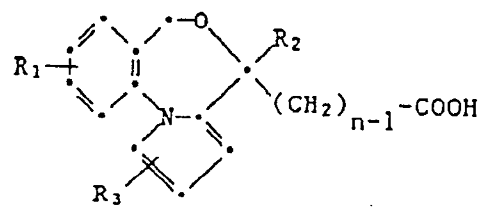

(IX)

wherein n represents the integer 1 or 2 ; $R_1$ represents hydrogen, lower alkyl, halogen, trifluoromethyl, hydroxy or lower alkoxy ; $R_2$ represents hydrogen or lower alkyl ; and $R_3$ represents hydrogen, lower alkyl or halogen, the radicals designated "lower" having a maximum of 7 carbon atoms, for the preparation of compounds of the general formula I according to claim 1.

10. The use of compounds of the general formula VI

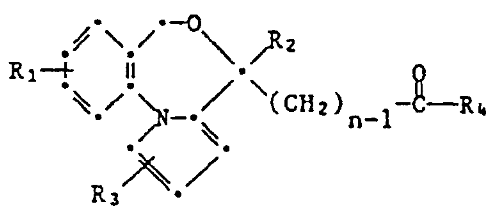

(VI)

wherein n represents the integer 1 or ; $R_1$ represents hydrogen, lower alkyl, halogen, trifluoromethyl, hydroxy or lower alkoxy ; $R_2$ represents hydrogen or lower alkyl ; $R_3$ represents hydrogen, lower alkyl or halogen ; $R_4$ represents N-lower alkylpiperazino, N-phenylpiperazino that is unsubstituted or substituted in the phenyl moiety by halogen or by lower alkyl, or piperidino or substituted piperidino selected from

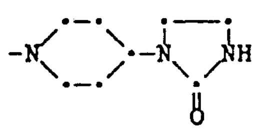 , 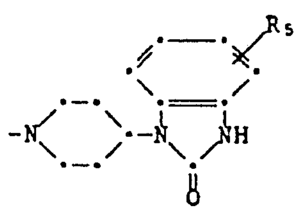 , 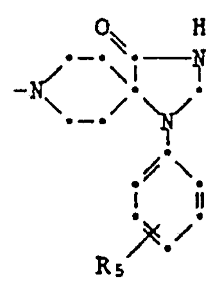 ,

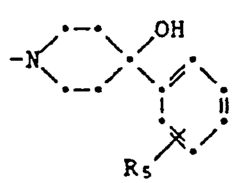 and 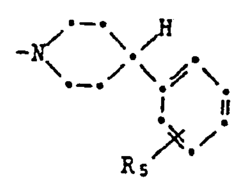 ,

in which $R_5$ represents hydrogen, halogen or lower alkyl, the radicals designated "lower" having a maximum of 7 carbon atoms, for the preparation of compounds of formula I according to claim 1.

## Revendications

### Revendications pour les Etats contractants : BE, CH, DE, FR, GB, IT, LI, LU, NL, SE

1. Composés de formule générale I

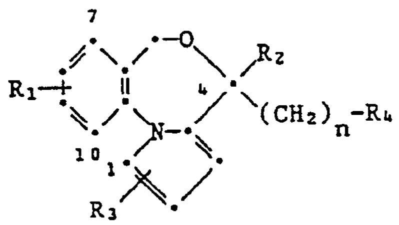

(I),

où n représente un nombre entier 1 ou 2 ; $R_1$ représente l'hydrogène, un alkyle inférieur, un halogène, le trifluorométhyle, un hydroxy ou un alcoxy inférieur ; $R_2$ est l'hydrogène ou un alkyle inférieur ; $R_3$ représente l'hydrogène, un alkyle inférieur ou un halogène ; $R_4$ représente un N-alkyle inférieur pipérazino ou un N-phénylpipérazino non substitué ou substitué dans le reste phényle par un halogène ou un alkyle inférieur, un pipéridino ou un pipéridino susbtitué parmi

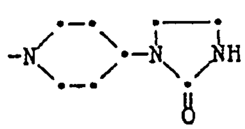

,

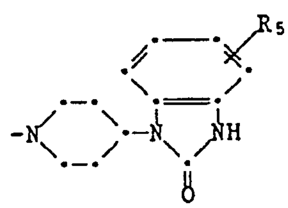

,

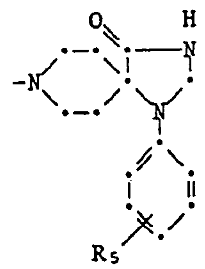

,

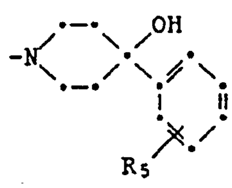

et

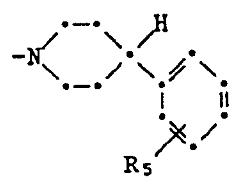

,

où $R_5$ représente l'hydrogène, un halogène ou un alkyle inférieur ; et leurs sels, les restes désignés d "inférieurs" présentant au plus 7 atomes de carbone.

2. Composés de formule II

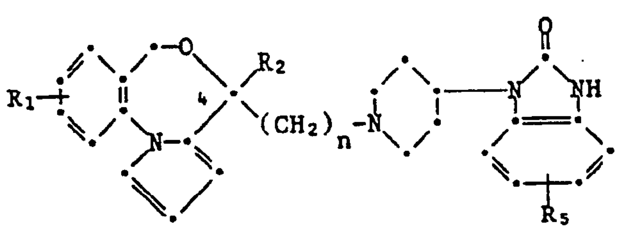

(II),

où $R_1$ représente l'hydrogène, un alkyle inférieur, un halogène, le trifluorométhyle, un hydroxy ou un alcoxy inférieur ; $R_2$ représente l'hydrogène ou un alkyle inférieur ; n représente un nombre entier 1 ou 2 ; et $R_5$ représente l'hydrogène, un halogène ou un alkyle inférieur, et leurs sels pharmaceutiquement acceptables ; Les restes désignés d "inférieurs" présentant au plus 7 atomes de carbone.

3. Composés de formule II montrée dans la revendication 2, où n représente un nombre entier 1 ou 2 ; $R_2$ représente un alkyle inférieur à chaîne droite ayant 1 à 4 atomes de carbone ; $R_1$ et $R_5$ représentent l'hydrogène ou un halogène et leurs sels pharmaceutiquement acceptables.

4. Composés de formule II montrée dans la revendication 2, où $R_1$ et $R_5$ représentent l'hydrogène ; $R_2$ représente le méthyle ; et n est égal à 1 et leurs sels pharmaceutiquement acceptables.

5. La 1,3-dihydro-1-{1-[(4-méthyl-4H,6H-pyrrolo[1,2-a][4,1]-benzoxazépine-4-yl)-méthyl]-4-pipéridinyl}-2H-benzimidazol-2-one et ses sels pharmaceutiquement acceptables.

6. Préparations pharmaceutiques contenant des composés selon les revendications 1 à 5 en association avec un support pharmaceutique.

7. Les composés définis dans les revendications 1 à 5 destinés à être utilisés dans un procédé pour le traitement thérapeutique du corps humain ou animal.

8. Les composés définis dans les revendications 1 à 5 destinés à être utilisés comme antidiarrhéique.

9. Les composés définis dans les revendications 1 à 5 destinés à être utilisés comme inhibiteur de la sécrétion gastro-intestinale.

10. Utilisation des composés définis dans les revendications 1 à 5 pour la préparation de compositions pharmaceutiques.

11. Procédé pour la préparation des composés de formule I définis dans la revendication 1 et leurs sels, caractérisé :

a) en ce que l'on condense une amine de formule III

$$R_4\text{-H} \quad (III)$$

où $R_4$ a la signification indiquée dans la revendication 1 et où H est lié à l'atome d'azote du groupe $R_4$, avec un composé de formule IV

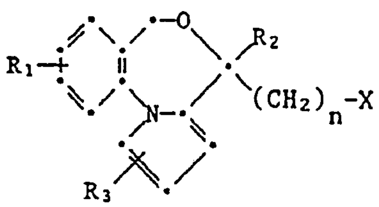

(IV),

où n, $R_1$, $R_2$ et $R_3$ ont la signification indiquée pour la formule I et X représente un groupe hydroxy estérifié réactif ou avec un composé de formule V

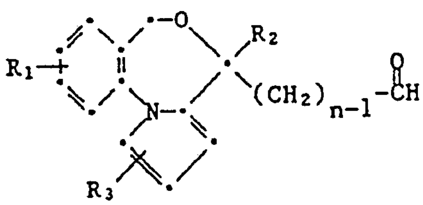

(V),

où n, $R_1$, $R_2$ et $R_3$ ont la signification indiquée dans la revendication 1 pour la formule I, dans les conditions d'une amination par voie de réduction ; ou

b) en ce que l'on réduit un composé de formule VI

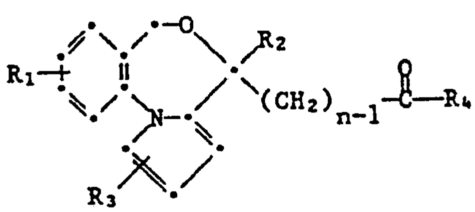

(VI),

où $R_1$, $R_2$, $R_3$, $R_4$ et n ont la signification indiquée dans la revendication 1 pour la formule I et, si désiré, en protégeant temporairement, dans tous ces procédés, un groupe réactif gênant, et en isolant le composé de formule I obtenu ; et, si désiré, en transformant le composé de formule I obtenu en un autre composé de formule I conforme à l'invention et/ou, si désiré, en transformant le composé de formule I obtenu en son sel ou le sel obtenu en un composé libre ou en un autre sel et/ou, si désiré, en séparant le mélange d'isomères ou de racémates obtenu en isomères ou racémates individuels et/ou, si désiré, en séparant les racémates obtenus en antipodes optiques.

12. Les composés obtenus selon le procédé de la revendication 11.

13. Composés de formule générale IV

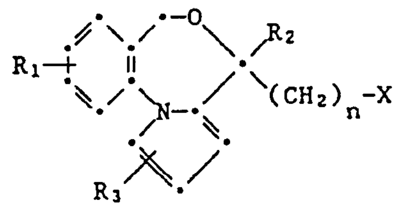 (IV),

où n est un nombre entier 1 ou 2 ; $R_1$ représente l'hydrogène, un alkyle inférieur, un halogène, le trifluorométhyle, un hydroxy ou un alcoxy inférieur ; $R_2$ représente l'hydrogène ou un alkyle inférieur ; $R_3$ est l'hydrogène, un alkyle inférieur ou un halogène ; et X représente l'hydroxy, les restes désignés d "inférieurs" présentant au plus 7 atomes de carbone.

14. Composés de formule générale V

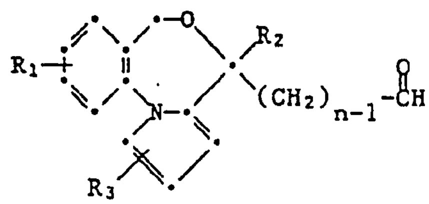 (V),

où n est un nombre entier 1 ou 2 ; $R_1$ représente l'hydrogène, un alkyle inférieur, un halogène, le trifluorométhyle, un hydroxy ou un alcoxy inférieur ; $R_2$ est l'hydrogène ou un alkyle inférieur ; et $R_3$ est l'hydrogène, un alkyle inférieur ou un halogène, les restes désignés d "inférieurs" présentant au plus 7 atomes de carbone.

15. Composés de formule générale IX

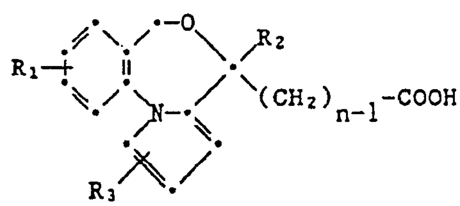 (IX),

où n est un nombre entier 1 ou 2 ; $R_1$ représente l'hydrogène, un alkyle inférieur, un halogène, le trifluorométhyle, un hydroxy ou un alcoxy inférieur ; $R_2$ est l'hydrogène ou un alkyle inférieur ; et $R_3$ est l'hydrogène, un alkyle inférieur ou un halogène, les restes désignés d "inférieurs" présentant au plus 7 atomes de carbone.

16. Composés de formule générale VI

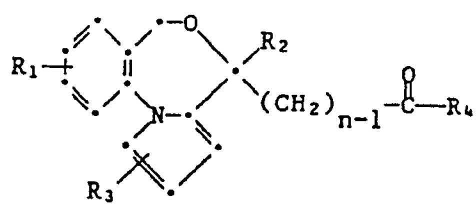 (VI),

où n représente un nombre entier 1 ou 2 ; $R_1$ représente l'hydrogène, un alkyle inférieur, un halogène, le trifluorométhyle, un hydroxy ou un alcoxy inférieur ; $R_2$ est l'hydrogène ou un alkyle inférieur ; $R_3$ représente l'hydrogène, un alkyle inférieur ou un halogène ; $R_4$ représente un N-alkyle inférieur pipérazino ou un N-phénylpipérazino non substitué ou substitué dans le reste phényle par un halogène ou un alkyle inférieur, un pipéridino ou un pipéridino substitué choisi parmi

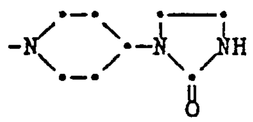 , 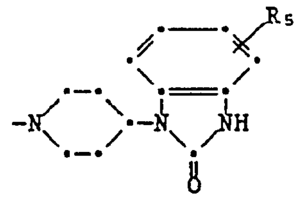 , 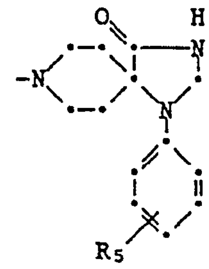 ,

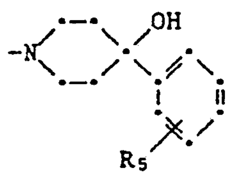 et 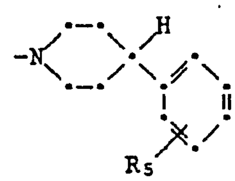

où $R_5$ représente l'hydrogène, un halogène ou un alkyle inférieur, les restes désignés d "inférieurs" présentant au plus 7 atomes de carbone.

**Revendications pour les Etats contractants : AT, ES, GR**

1. Procédé pour la préparation des composés de formule générale I

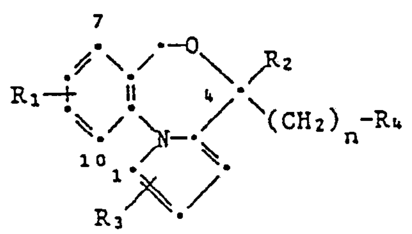 (I),

où n représente un nombre entier 1 ou 2 ; $R_1$ représente l'hydrogène, un alkyle inférieur, un halogène, le trifluorométhyle, un hydroxy ou un alcoxy inférieur ; $R_2$ est l'hydrogène, un alkyle inférieur ; $R_3$ représente l'hydrogène, un alkyle inférieur ou un halogène ; $R_4$ représente un N-alkyle inférieur pipérazino ou un N-phénylpipérazino non substitué ou substitué dans le reste phényle par un halogène ou un alkyle inférieur, un pipéridino ou un pipéridino substitué choisi parmi

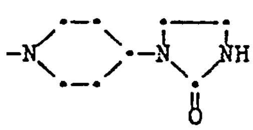 , 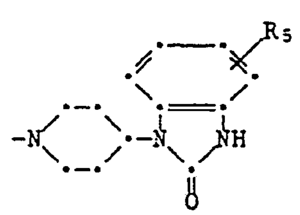 , 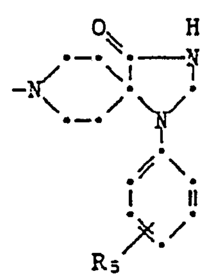 ,

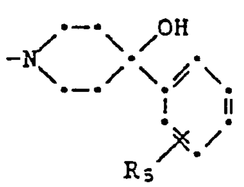 ,et 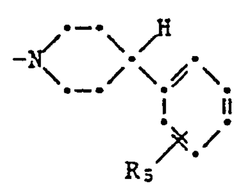 ,

où $R_5$ représente l'hydrogène, un halogène ou un alkyle inférieur ; et de leurs sels, les restes désignés d "inférieurs" présentant au plus 7 atomes de carbone, caractérisé

a) en ce que l'on condense une amine de formule III

$$R_4\text{-H} \quad \text{(III)}$$

où $R_4$ a la signification indiquée ci-dessus et où H est lié à l'atome d'azote du groupe $R_4$, avec un composé de formule IV

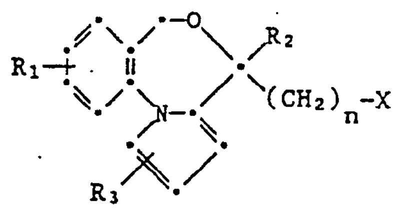

(IV),

où n, $R_1$, $R_2$ et $R_3$ ont la signification indiquée pour la formule I et X représente un groupe hydroxy estérifié réactif ou avec un composé de formule V

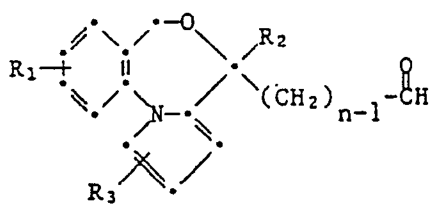

(V),

où n, $R_1$, $R_2$ et $R_3$ ont la signification indiquée pour la formule I, dans les conditions d'une amination par voie de réduction ; ou

b) en ce que l'on réduit un composé de formule VI

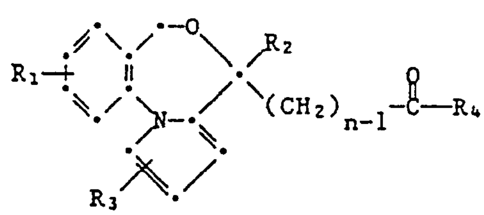

(VI),

où $R_1$, $R_2$, $R_3$, $R_4$ et n ont la signification indiquée pour la formule I et, si désiré, en protégeant temporairement, dans tous ces procédés, un groupe réactif gênant, et en isolant le composé de formule I obtenu ; et, si désiré, en transformant le composé de formule I obtenu en un autre composé de formule I conforme à l'invention et/ou, si désiré, en transformant le composé de formule I obtenu en son sel ou le sel obtenu en un composé libre ou en un autre sel et/ou, si désiré, en séparant le mélange d'isomères ou de racémates obtenu en isomères ou racémates individuels et/ou, si désiré, en séparant les racémates obtenus en antipodes optiques.

2. Procédé selon la revendication 1 pour la préparation des composés de formule II

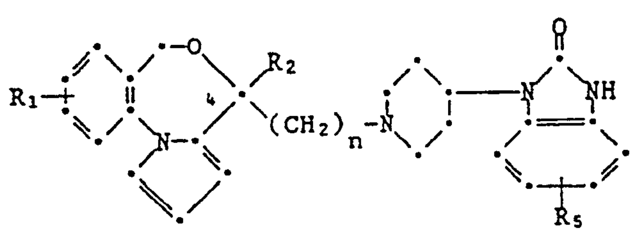

(II),

où $R_1$ représente l'hydrogène, un alkyle inférieur, un halogène, le trifluorométhyle, un hydroxy ou un alcoxy inférieur ; $R_2$ représente l'hydrogène ou un alkyle inférieur ; n représente un nombre entier 1 ou 2 ; et $R_5$ représente l'hydrogène, un halogène ou un alkyle inférieur ; et de leurs sels pharmaceutiquement acceptables, les restes désignés d "inférieurs" présentant au plus 7 atomes de carbone.

3. Procédé selon la revendication 1 pour la préparation des composés de formule II montrée dans la revendication 2 où n représente un nombre entier 1 ou 2 ; $R_2$ représente un alkyle inférieur à chaîne droite ayant 1 à 4 atomes de carbone ; $R_1$ et $R_5$ représentent l'hydrogène ou un halogène, et de leurs sels pharmaceutiquement acceptables.

4. Procédé selon la revendication 1 pour la préparation des composés de formule II montrée dans la revendication 2 où $R_1$ et $R_5$ représentent l'hydrogène ; $R_2$ représente le méthyle ; et n est égal à 1, et de leurs sels pharmaceutiquement acceptables.

5. Procédé selon la revendication 1 pour la préparation de la 1,3-dihydro-1-{1-[(4-méthyl-4H,6H-pyrrolo[1,2-a][4,1]-benzoxazépine-4-yl)-méthyl]-4-pipéridin-yl}-2H-benzimidazol-2-one et de ses sels pharmaceutiquement acceptables.

6. Procédé pour la préparation d'une composition pharmaceutique, caractérisé par la mise en oeuvre d'une substance active conforme à l'invention selon l'une des revendications 1 à 5 avec un support pharmaceutique.

7. Utilisation des composés de formule générale IV

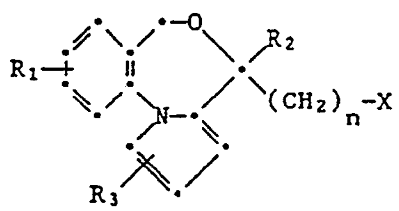

(IV),

où n est un nombre entier 1 ou 2 ; $R_1$ représente l'hydrogène, un alkyle inférieur, un halogène, le trifluorométhyle, un hydroxy ou un alcoxy inférieur ; $R_2$ représente l'hydrogène ou un alkyle inférieur ; $R_3$ est l'hydrogène, un alkyle inférieur ou un halogène ; et X représente l'hydroxy, les restes désignés d "inférieurs" présentant au plus 7 atomes de carbone, pour la préparation des composés de formule générale I selon la revendication 1.

8. Utilisation des composés de formule générale V

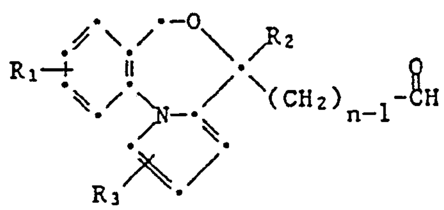

(V),

où n est un nombre entier 1 ou 2 ; $R_1$ représente l'hydrogène, un alkyle inférieur, un halogène, le trifluorométhyle, un hydroxy ou un alcoxy inférieur ; $R_2$ est l'hydrogène ou un alkyle inférieur ; et $R_3$ est l'hydrogène, un alkyle inférieur ou un halogène, les restes désignés d "inférieurs" présentant au plus 7 atomes de carbone, pour la préparation des composés de formule générale I selon la revendication 1.

9. Utilisation des composés de formule générale IX

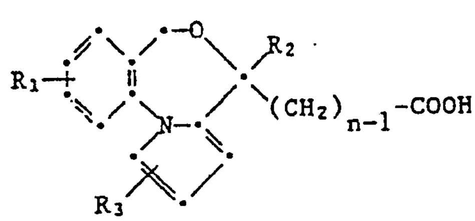

(IX),

où n est un nombre entier 1 ou 2 ; $R_1$ représente l'hydrogène, un alkyle inférieur, un halogène, le trifluorométhyle, un hydroxy ou un alcoxy inférieur ; $R_2$ est l'hydrogène ou un alkyle inférieur ; et $R_3$ est l'hydrogène, un alkyle inférieur ou un halogène, les restes désignés d "inférieurs" présentant au plus 7 atomes de carbone, pour la préparation des composés de formules générale I selon la revendication 1.

10. Utilisation des composés de formule générale VI

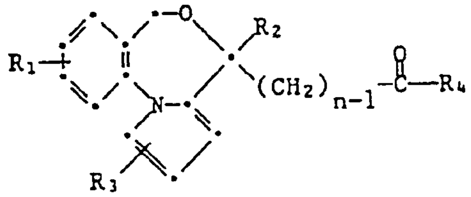 (VI),

où n représente un nombre entier 1 ou 2 ; $R_1$ représente l'hydrogène, un alkyle inférieur, un halogène, le triflorométhyle, un hydroxy ou un alcoxy inférieur ; $R_2$ est l'hydrogène ou un alkyle inférieur ; $R_3$ représente l'hydrogène, un alkyle inférieur ou un halogène ; $R_4$ repréente un N-alkyle inférieur pipérazino ou un N-phénylpipérazino non substitué ou substitué dans le reste phényle par un halogène ou un alkyle inférieur, un pipéridino ou un pipéridino substitué choisi parmi

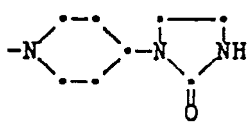 , 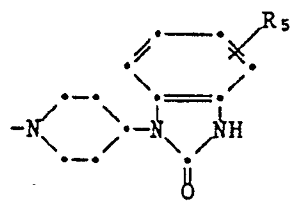 , 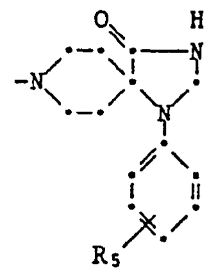 ,

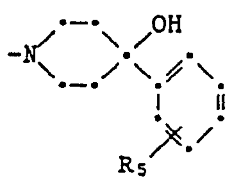 et 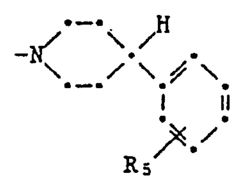 ,

où $R_5$ représente l'hydrogène, un halogène ou un alkyle inférieur, les restes désignés d "inférieurs" présentant au plus 7 atomes de carbone, pour la préparation des composés de formule générale I selon la revendication 1.